# EUROPEAN PATENT APPLICATION

(11) **EP 2 314 713 A1**
(43) Date of publication of application: **27.04.2011**
(21) Application number: 09773480.0
(22) Date of filing: 30.06.2009
(51) Int. Cl.: C12Q 1/68, C12M 1/00, C12N 15/09, G01N 33/53

(54) **OLIGONUCLEOTIDE PROBE, AND USE THEREOF**

(30) Priority: 01.07.2008 JP 2008172526; 06.11.2008 JP 2008285966
(71) Applicant: National University Corporation Nagoya University, Aichi 464-8601 (JP); NGK Insulators, Ltd., Nagoya-shi, Aichi 467-8530 (JP)
(72) Inventor: ASANUMA Hiroyuki, Nagoya-shi Aichi 464-8601 (JP); LIANG Xingguo, Nagoya-shi Aichi 464-8601 (JP); KASHIDA Hiromu, Nagoya-shi Aichi 464-8601 (JP); YOSHIDA Yasuko, Nagoya-shi Aichi 467-8530 (JP); TAKASE Tomokazu, Nagoya-shi Aichi 467-8530 (JP); NIWA Kousuke, Nagoya-shi Aichi 467-8530 (JP)
(74) Representative: Paget, Hugh Charles Edward
(86) International application number: PCT/JP2009/061980
(87) International publication number: WO 2010/001902

(57) **Abstract**

The present teaching provides a fluorescent oligonucleotide probe having a high degree of design flexibility and wide applicability, as well as the use thereof. This is an oligonucleotide probe capable of forming a stem and loop, comprising at least one fluorophore located between adjacent nucleotides in the stem and is linked to a unit represented by Formula (1) and at least one quencher located at a site capable of pairing up with the at least one fluorophore located between the adjacent nucleotides in the stem and is linked to a unit represented by Formula (2). (In the formulae, X represents the fluorophore, Y represents the quencher, R1 represents an optionally substituted C₂ or C₃ alkylene chain, R2 represents an optionally substituted C₀₋₂ alkylene chain, and Z represents a direct bond or linker.)

SEQUENCE LISTING

<110>  National University Corporation Nagoya University
       NGK INSULATORS, LTD.

<120>  Oligonucleotides and the Use Thereof

<130>  HP/FP6741664

<140>  EP09773480.0
<141>  2009-06-30

<150>   PCT/JP2009/061980
<151>   2009-06-30

<150>   2008-172526
<151>   2008-07-01

<150>   2008-285966
<151>   2008-11-06

<160>  8

<170>  PatentIn version 3.3

<210>  1
<211>  13
<212>  DNA
<213>  Artificial

<220>
<223>  Synthetic oligonucleotide


<220>
<221>  misc_feature
<222>  (7)..(7)
<223>  Artificial unit

<400>  1
ggtatcngca atc                                                          13


<210>  2
<211>  13
<212>  DNA
<213>  Artificial

<220>
<223>  Synthetic oligonucleotide


<220>
<221>  misc_feature
<222>  (7)..(7)
<223>  Artificial unit

<400>  2
gattccngat acc                                                          13


<210>  3
<211>  25
<212>  DNA
<213>  Artificial

<220>
<223>  Synthetic oligonucleotide

<220>
<221>  misc_feature
<222>  (3)..(3)
<223>  Artificial  unit

<220>
<221>  misc_feature
<222>  (23)..(23)
<223>  Artificial  unit

<400>  3
tgngtccttg agaaagggcg acnca                                          25


<210>  4
<211>  25
<212>  DNA
<213>  Artificial

<220>
<223>  Synthetic oligonucleotide


<220>
<221>  misc_feature
<222>  (4)..(4)
<223>  Artificial unit

<220>
<221>  misc_feature
<222>  (22)..(22)
<223>  Artificial unit

<400>  4
tggntccttg agaaagggcg ancca                                          25


<210>  5
<211>  23
<212>  DNA
<213>  Artificial

<220>
<223>  Synthetic oligonucleotide

<400>  5
tggtccttga gaaagggcga cca                                            23


<210>  6
<211>  18
<212>  DNA
<213>  Artificial

<220>
<223>  Synthetic oligonucleotide

<400>  6
gccctttctc aaggacca                                                  18


<210>  7
<211>  27
<212>  DNA
<213>  Artificial

<220>
<223>  Synthetic oligonucleotide

<220>
<221>  misc_feature
<222>  (2)..(2)
<223>  Artificial unit

<220>
<221>  misc_feature
<222>  (5)..(5)
<223>  Artificial unit

<220>
<221>  misc_feature
<222>  (23)..(23)
<223>  Artificial unit

<220>
<221>  misc_feature
<222>  (26)..(26)
<223>  Artificial unit

<400>  7
tnggntcctt gagaaagggc ganccna                                          27


<210>  8
<211>  18
<212>  DNA
<213>  Artificial

<220>
<223>  Synthetic oligonucleotide

<400>  8
tggtcgccct ttctcaag                                                    18

## Description

### TECHNICAL FIELD

The present invention relates to an oligonucleotide probe and a use thereof.

### BACKGROUND ART

Oligonucleotide probes include those that self-generate fluorescence. These fluorescence self-generating probes include those called molecular beacons. Molecular beacons are oligonucleotides that produce a detectable fluorescent signal on hybridization with a target nucleic acid (Patent Document 1). Molecular beacons are artificial oligonucleotides having a stem-loop structure with a fluorophore and a quencher (fluorescence quenching substance) at either end of the oligonucleotide. When the molecular beacon is in a single-stranded state, the stem region normally forms a double strand, bringing the fluorophore and quencher introduced at either end into proximity with one another so that the fluorescence of the fluorophore is quenched by the quencher. However, on hybridization with an oligonucleotide complementary to the base sequence of the loop region of the probe, however, the fluorophore and quencher are pulled apart, and a fluorescent signal is produced. Using such a molecular beacon as a probe, hybridization with the target nucleic acid can be detected without fluorescent labeling of the target.

Conventionally, such molecular beacons have mainly been used in liquid-phase reactions, such as for detecting amplification products in real-time PCR and the like.

Patent Document 1: Japanese Patent Application Publication No. 2004-329209

Molecular beacons quench by forming a stable stem at temperatures sufficiently lower than the melting temperature of the stem, and emit fluorescence based on the fluorophore when the stem is opened by hybridization with the target nucleic acid. However, emission strength and interaction with the target nucleic acid are greatly affected by temperature. That is, when the target nucleic acid is present at a temperature lower than the melting temperature of the stem, the molecular beacon forms a double strand with the target nucleic acid and emits fluorescence, but if the temperature rises, the molecular beacon separates from the target nucleic acid, quenching the fluorescence. Therefore, the melting temperature of the stem must be sufficiently high so that a stable quenched state can be formed in order to maintain the quenched state in the absence of the target nucleic acid, and reduce background. At the same time, the melting temperature of the target nucleic acid with the molecular beacon is preferably as high as possible and close to the melting temperature of the stem so that hybridization between the target nucleic acid and the molecular beacon can be detected more reliably.

However, because the overall length of the molecular beacon is somewhat limited, if the stem is lengthened in order to raise its melting temperature, probe design flexibility is reduced and there is a risk of reduced detection specificity for the target nucleic acid.

In particular, when considering a molecular beacon array of a plurality of molecular beacons immobilized on a carrier, with each beacon corresponding to a target nucleic acid to be detected, it is desirable from the standpoint of ensuring detection accuracy to not only raise the melting temperatures of the stems, but also to design the beacons so that they can hybridize with sufficiently long target nucleic acids. For the reasons mentioned above, however, it has been difficult to achieve both these goals simultaneously in a molecular beacon.

That is, with conventional molecular beacons it has been extremely difficult to control background while ensuring specificity with the target nucleic acid.

### SUMMARY OF INVENTION

It is therefore an object of the present teaching to provide a fluorescent oligonucleotide probe having a higher degree of design flexibility and wide applicability, along with a use thereof.

The inventors in this case discovered that more reliable quenching and improved thermal stability of the stem could be achieved by arranging the fluorophore and quencher so that they can form a stable stack structure when brought close to one another when the stem is formed, rather than making quenching and fluorescence heavily dependent on proximity effect caused by stem formation and separation effect caused by double-strand formation with the target nucleic acid. They also discovered that there are cases in which fluorescence can be amplified by causing the fluorophore to be incorporated into the double strand with the target nucleic acid when a double strand is formed with a target nucleic acid. The inventors perfected the present teaching based on these findings. The following means are provided by the disclosures of the present Specification.

The disclosures of the present Specification provide an oligonucleotide probe capable of forming a stem and loop. The oligonucleotide probe comprises: at least one fluorophore that is located between adjacent nucleotides in the stem and is linked to a unit represented by the following Formula (1): 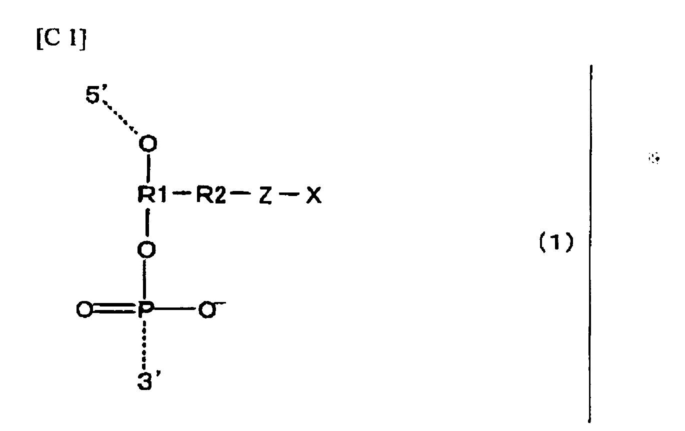 (in the formula, X represents the fluorophore, R1 represents an optionally substituted C₂ or C₃ alkylene chain, R2 represents an optionally substituted C₀₋₂ alkylene chain, and Z represents a direct bond or linker); and at least one quencher that is located at a site capable of pairing up with the at least one fluorophore located between the adjacent nucleotides in the stem and is linked to a unit represented by the following Formula (2): 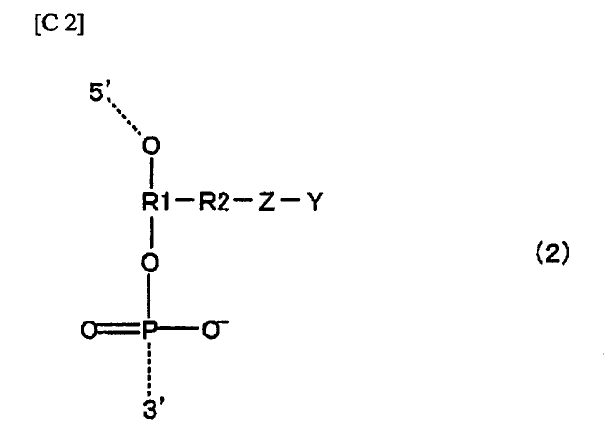 (in the formula, Y represents the quencher, R1 represents an optionally substituted C₂ or C₃ alkylene chain, R2 represents an optionally substituted C₀₋₂ alkylene chain, and Z represents a direct bond or linker). In Formulae (1) and (2), the phosphate group (PO₃⁻) in the phosphodiester bond may be of the nondissociative type (PO₃H).

In the oligonucleotide probe disclosed herein, the fluorophore is preferably located within a nucleotide strand constituting a base sequence that hybridizes specifically with a target nucleic acid. In the oligonucleotide probe disclosed herein, this fluorophore is preferably any one selected from the group consisting of cyanine dyes, merocyanine dyes, condensed aromatic ring dyes and xanthene dyes, and the quencher is preferably any one selected from the group consisting of azo dyes. More preferably, the fluorophore is any one selected from the group consisting of Cy3, Cy5, thiazole orange, oxazole yellow, perylene, fluorescein, rhodamine, tetramethyl rhodamine and Texas red, and the quencher is any one selected from methyl red, azobenzene and methylthioazobenzene.)

It is also desirable that the fluorophore be a condensed aromatic ring dye while the quencher is anthraquinone or its derivative, and more desirable that the fluorophore by perylene while the quencher is anthraquinone.

The oligonucleotide probe disclosed herein is preferably one capable of forming one stem and one loop.

The disclosures of the present Specification provide an immobilized probe body for detecting a target nucleic acid, comprising: a solid-phase carrier; and any of the oligonucleotide probes described above supported on the solid phase carrier and having a base sequence capable of hybridizing specifically with at least part of the target nucleic acid. In the immobilized probe body disclosed herein, the solid-phase carrier is preferably a plate. The immobilized probe body disclosed herein is also preferably provided with an array of a plurality of oligonucleotide probes capable of detecting a plurality of the target nucleic acids, respectively. The immobilized probe body disclosed herein is preferably used for detecting SNPs or gene variants.

The disclosures of the present Specification provide a method for detecting a target nucleic acid, which is a method for using the oligonucleotide probe disclosed herein having a base sequence capable of hybridizing specifically with at least part of the target nucleic acid, to detect a signal based on the fluorophore of the oligonucleotide probe.

In the detection method disclosed herein, it is desirable to use an immobilized body having a solid-phase carrier supporting a plurality of the oligonucleotide probes capable of pairing and hybridizing with a plurality of target nucleic acids.

### BRIEF DESCRIPTION OF THE DRAWINGS

Fig. 1 gives an outline of one example of the oligonucleotide probe disclosed herein.
Fig. 2 shows one example of the oligonucleotide probe disclosed herein as a molecular beacon.
Fig. 3A shows the absorption spectra of various oligonucleotides provided with thiazole orange.
Fig. 3B shows the fluorescent spectra of various oligonucleotides provided with thiazole orange.
Fig. 4 shows the fluorescence spectrum of an oligonucleotide provided with perylene.

### DESCRIPTION OF EMBODIMENTS

### Preferred Aspects of Invention

The disclosures of the present Specification relate to a fluorescent oligonucleotide probe, and also to an immobilized body of this probe and to a method for detecting a target nucleic acid using this probe. With the oligonucleotide probe disclosed herein, when no target nucleic acid is present a fluorophore and a quencher form a stable stack structure on the stem, increasing thermal stability and stably quenching fluorescence. When a target nucleic acid is present, the oligonucleotide probe hybridizes with the target nucleic acid at a nucleotide strand (hereunder sometimes called a probe sequence) constituting a base sequence in the probe that hybridizes specifically with the target nucleic acid. As a result, the stem is broken and the fluorophore is no longer quenched by the quencher, and emits fluorescence.

With the oligonucleotide probe disclosed herein, the stem can more easily have a high melting temperature because it is thermally stabilized by the fluorophore and the quencher, and it is possible to select longer or more diverse target nucleic acids (probe sequences). As a result, an oligonucleotide probe is provided that has wide applicability and can be designed with a high degree of flexibility according to the type of target nucleic acid and the hybridization conditions.

Moreover, immobilization on an immobilized probe body is easier because the fluorophore and quencher do not need to be located at the ends of the probe as in a conventional molecular beacon, making it possible to avoid or control the effects of immobilization on quenching and fluorescence.

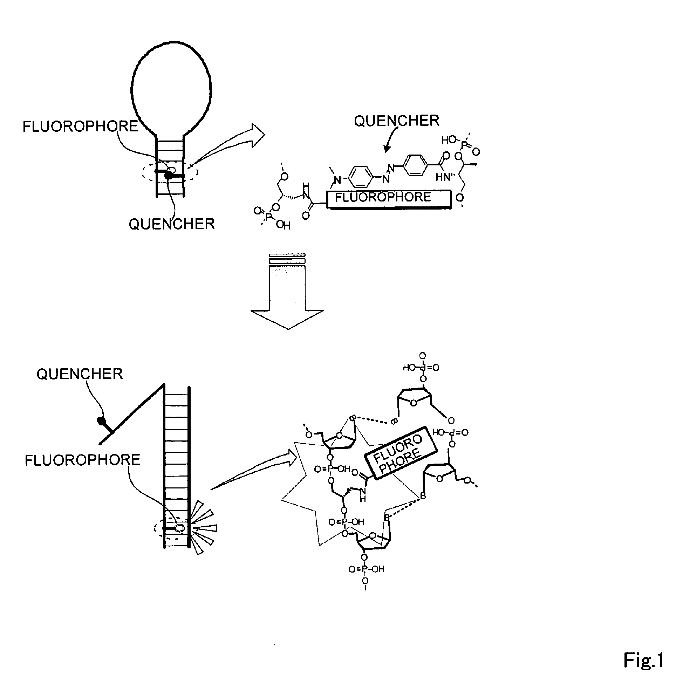

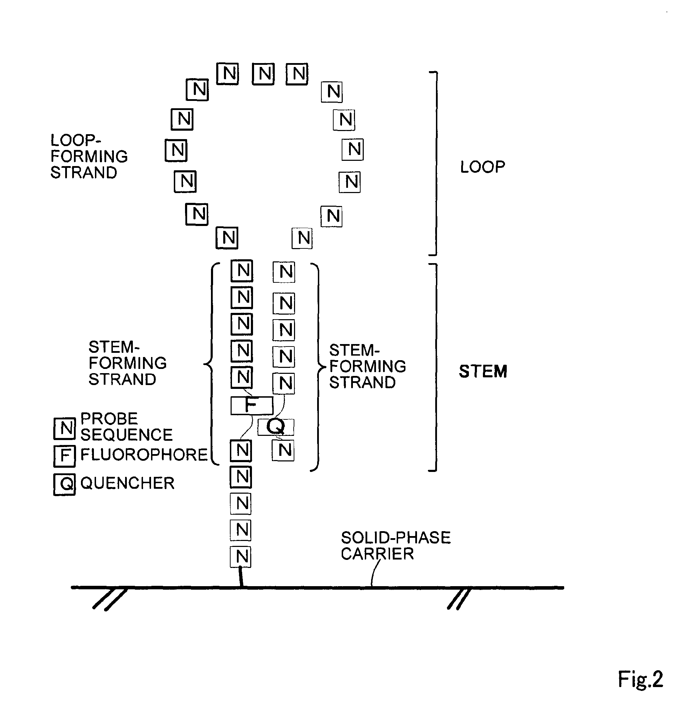

A fluorescent oligonucleotide probe, an immobilized body thereof and a method for detecting a target nucleic acid are described below with reference to the appropriate drawings as embodiments of the means disclosed herein. Fig. 1 shows an action of a molecular beacon-type probe as one example of the fluorescent oligonucleotide probe disclosed herein. Fig. 2 shows one example of a molecular beacon-type probe provided on a solid-phase carrier.

Herein, an oligonucleotide may be one having natural or artificial bases, nucleotides or nucleosides that allow it to provide the function of hybridization with natural, naturally-derived or artificial DNA or RNA to be detected. For purposes as a probe, an oligonucleotide generally means roughly 10mers to 100mers, but the number is not especially limited and those that are longer than 100mers are also included in the oligonucleotide probe of the present Specification. Herein, the target nucleic acid is not particularly limited as to derivation or the like, and may be natural, naturally-derived or artificial DNA (single or double-stranded) or RNA (single or double-stranded), or a chimera or hybrid of these. Naturally-derived DNA means DNA prepared by genetic engineering, enzyme synthesis or chemical synthesis based on the base sequence of natural DNA.

### (Oligonucleotide probe)

As shown in Figs. 1 and 2, the oligonucleotide probe disclosed herein is capable of forming a stem and loop, and is provided with a fluorophore and quencher on the stem.

The oligonucleotide probe disclosed in these Specification may be any capable of forming at least one stem and at least one loop, and may also form a plurality of stems and a plurality of loops. Thus, it may be capable of forming a so-called molecular beacon-type probe provided with one stem and one loop as shown in Figs. 1 and 2, or may be capable of forming a plurality of stems and a plurality of loops of tRNA or similar structures. As discussed below, the oligonucleotide probe disclosed herein can provide adequate detection specificity even if it takes the form of the simplest molecular beacon.

The fact that the oligonucleotide probe disclosed herein can form a stem and loop means that it is provided with one pair of stem-forming strands for forming pairs to form at least one stem, and is also provided with a loop-forming strand that forms a loop by linking to the stem formed by the stem-forming strands. When stems or loops are simply referred to herein, it means the formed stems or loops.

The lengths of the stem-forming strands forming the stem are not particularly limited, and each of the stem-forming strands may have a strand length for forming at least 4 but no more than 9 base pairs. In the case of a molecular beacon probe, a strand length for forming at least 8 but no more than 40 or preferably at least 15 but no more than 30 base pairs is desirable. The stem in the oligonucleotide probe disclosed herein is not limited to being composed of both ends of the oligonucleotide strand. One of the stem-forming strands may consist of a part other than an end of the oligonucleotide probe while the other stem-forming strand is an end of the probe, or both stem-forming strands may be parts of the probe other than the ends.

The length of the loop strand for forming the loop is also not particularly limited, but may be roughly at least 5 but no more than 40. In the case of a molecular beacon, at least 8 but no more than 30 or preferably at least 10 but no more than 20 is desirable.

The oligonucleotide probe disclosed herein is provided with a fluorophore and a quencher each located between adjacent nucleotides of one of a pair of stem-forming strands on at least one stem. That is, neither the fluorophore nor the quencher is connected outside the 5' terminal or 3' terminal of the oligonucleotide probe. Even when located nearest the ends, the fluorophore and quencher are provided before the last nucleotides of the strands (that is, on the 3' side of the 5' terminal or the 5' side of the 3' terminal).

At least one fluorophore and one quencher are provided on one stem. The fluorophore and quencher do not necessarily need to be paired one on one as long as fluorescence is quenched when the stem is formed and turned on when the probe hybridizes with a target nucleic acid, but from the standpoint of hybridization with the target nucleic acid, a paired set of one fluorophore with one quencher is more desirable than a set of 3 or more consisting of two adjacent fluorophores with 1 or 2 or more quenchers. Because the fluorophore and quencher can at least form a strong 1:1 pair in this way, transfer of holes or electrons from the fluorophore to the quencher can operate more efficiently. This serves to strongly suppress background fluorescence when the beacon is closed. When a plurality of pairs of fluorescent dyes and quenchers is introduced into the stem, excited electrons (or holes) of fluorescent dye can move via base pairs to quenchers outside the pair, resulting in efficient quenching when the beacon is closed. This serves to improve the signal strength ratio.

When a plurality of fluorophores and quenchers is provided on a single stem, different kinds of fluorophores can be used, but it is desirable to use a combination of fluorophores emitting fluorescence that is detectable in the same wavelength band and quenched to the same degree. It is more desirable to use the same fluorophore.

When a plurality of fluorophores or a plurality of quenchers is provided on the same stem, 1 or 2 or more nucleotides are preferably present between adjacent fluorophores or adjacent quenchers. For example, the intervening nucleotides are not particularly limited but may be selected from the purine bases and/or pyrimidine bases, or may be artificially synthesized bases.

The fluorophore is located within the stem as discussed above, and is preferably positioned within the probe sequence of the oligonucleotide probe. This is because the fluorescence of certain fluorophores is enhanced if they intercalate within the double strand formed by hybridization with the target nucleic acid. This type of fluorophores will be described later.

There are no particular limitations on the types of bases in the fluorophore or quencher, or in the adjacent nucleotides, or in the distant nucleotides adjacent to these nucleotides.

The fluorophore is included as X in the unit represented by Formula (1) between nucleotides of the stem-forming strand. Fluorophore (X) is provided on the stem either as part of this unit or by being linked to a unit part other than Y. R1 in Formula (1) is an optionally substituted C₂ or C₃ alkylene chain. R2 is an optionally substituted C₀₋₂ alkylene chain. R2 is preferably bound to the second carbon atom from the 5' oxygen atom of the polyalkylene chain of R1. Z may be a direct bond or a linker with the fluorophore, without any particular limitations. Examples include NHCO-, NHCS-, CONH-, -O- and the like or groups containing these groups.

Examples of substituents in R1 and R2 above include C₁₋₂₀ or preferably C₁₋₁₀ or more preferably C₁₋₄ alkyl or alkoxy groups, which may unsubstituted or substituted by a person skilled in the art with halogen atoms, hydroxyl groups, amino groups, nitro groups or carboxyl groups; C₂₋₂₀ or preferably C₂₋₁₀ or more preferably C₂₋₄ alkenyl or alkynyl groups, which may be unsubstituted or substituted with halogen atoms, hydroxyl groups, amino groups, nitro groups, carboxyl groups or the like; and hydroxyl groups, halogen atoms, amino groups, nitro groups or carboxyl groups and the like. Other examples include hydroxyl groups, halogen atoms, amino groups, nitro groups or carboxyl groups. A substituent in R1 may be linked to any carbon atom in the alkylene chain, but as in the case of R2, is preferably linked to the second or third carbon atom from the 5' oxygen.

With respect to the paired fluorophore and quencher in the stem, the number of carbon atoms in the alkylene chains of R1 in Formula (1) and R1 in Formula (2) may be the same or different.

For example, the following are examples of the units represented by Formula (1) and Formula (2).

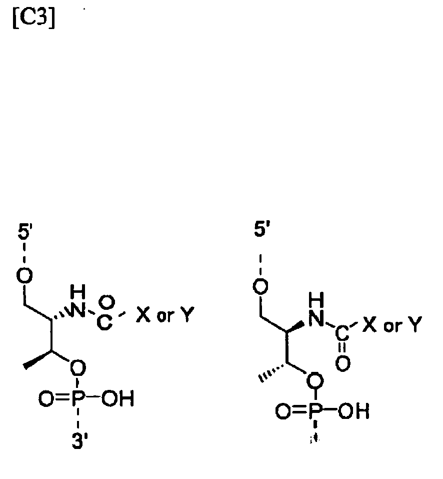

X or Y X or Y
R1 = C₃ alkylene group (substituted with methyl group)
R2 = no alkylene group
P=-NH-CO- 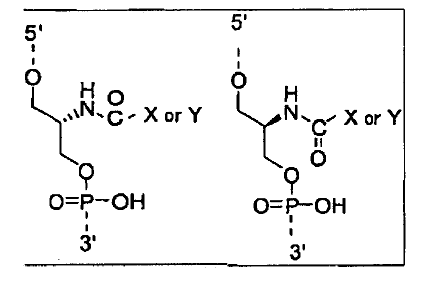 XorY X or Y
R1 C₃ alkylene group (unsubstituted)
R2 = no alkylene group
P = NH-CO- 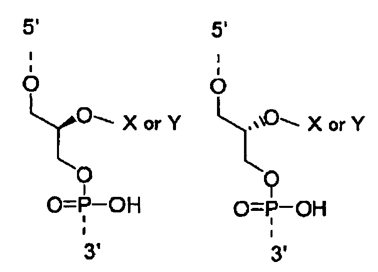 XorY X or Y
R1 = C₃ alkylene group (unsubstituted)
R2 = no alkylene group
P=-O- 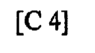 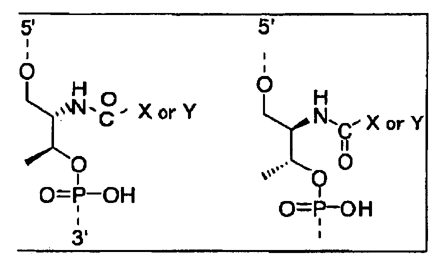 X or Y X or Y
R1 =C₂ alkylene group (substituted with methyl group)
R2 = C₁ alkylene group
P or Q = NH-CO- 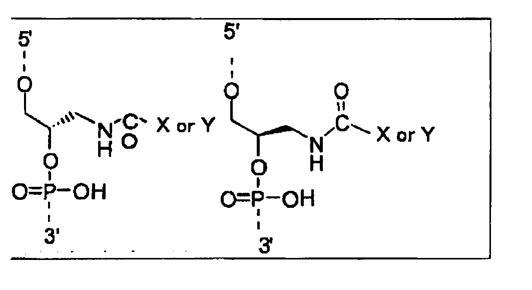 X or Y X or Y
R1 =C₂ alkylene group (unsubstituted)
R2 = C₁ alkylene group
P = -NH-CO- 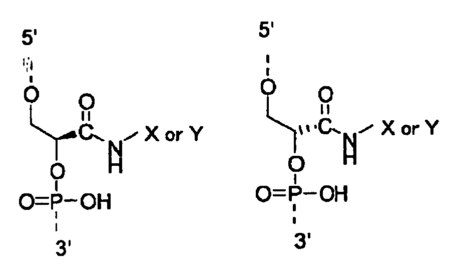 XorY X or Y
R1 = C₂ alkylene group (unsubstituted)
R2 = C₁ alkylene group
P =-CO-NH- 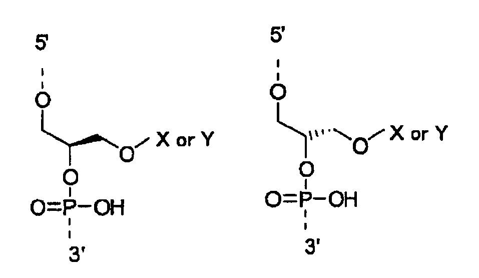 X or Y X or Y
R1 = C₂ alkylene group (unsubstituted)
R2 = C₁ alkylene group
P=-O- 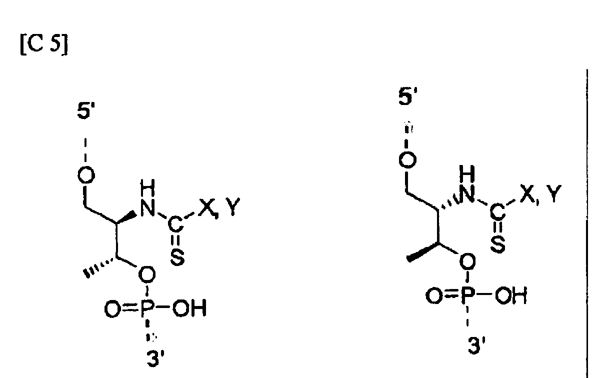 R1 =C₃ alkylene group (substituted with methyl group)
R2 = None
P = -NH-CS- 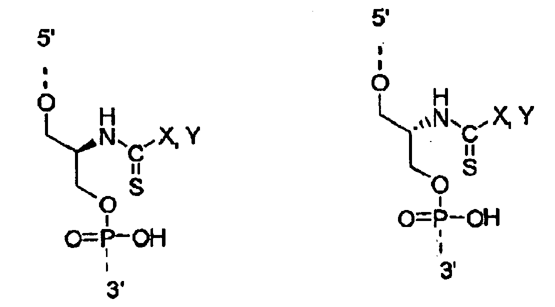 R1= C₃ alkylene group (unsubstituted)
R2 = None
P=-NH-CS- 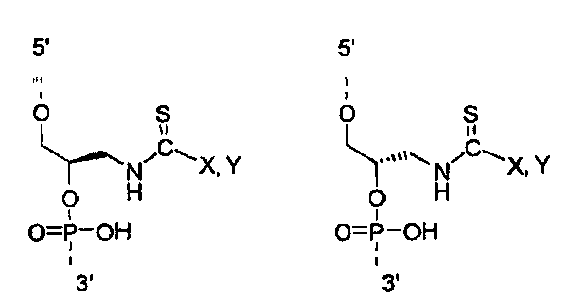 R1 =C₂ alkylene group (unsubstituted)
R2 = C₁ alkylene group
P = -NH-CS-

In Formula (1), X represents the fluorophore. Examples of fluorophores include cyanine dyes, merocyanine dyes, acridine dyes, coumarin dyes, ethidium dyes, flavin dyes, condensed aromatic ring dyes, xanthene dyes and the like, and these may be selected and used appropriately. Of these, a cyanine dye, coumarin dye, ethidium dye, condensed aromatic ring dye or xanthene dye can be used by preference. More preferably, the fluorophore is selected from the group consisting of Cy3, Cy5, thiazole orange, oxazole yellow, perylene, fluorescein, rhodamine, tetramethyl rhodamine and Texas red. Of these, thiazole orange and other cyanine dyes are especially preferred. The compound used as the fluorophore may be derivatized as necessary for purposes of linking to the unit represented by Formula (1), and an optionally substituted alkylene chain, alkenylene group or alkynylene group may also be provided between the fluorophore and the linking group for purposes of pairing with the quencher. There are no particular limits on which atoms of the fluorophore are linked to the unit represented by Formula (1).

Examples of fluorophores whose fluorescence is increased by intercalation include thiazole orange, oxazole orange, perylene and the like. These fluorophores are preferably intercalated by positioning them within the probe sequence. Depending on the fluorophore and the combination of R1 and R2, the fluorescent strength of the fluorophore may actually be diminished by intercalation, in which case it is desirable to design the beacon so that the quencher is intercalated instead of the fluorophore.

The following are examples of the fluorophore (X) linked in Formula (1). The various compounds in the examples are only examples, and any of the hydrogen atoms may be replaced with appropriate substituents. An optionally substituted alkylene group, alkenylene group or alkynylene group may also be interposed at the linkage (dotted line) with the linking group.

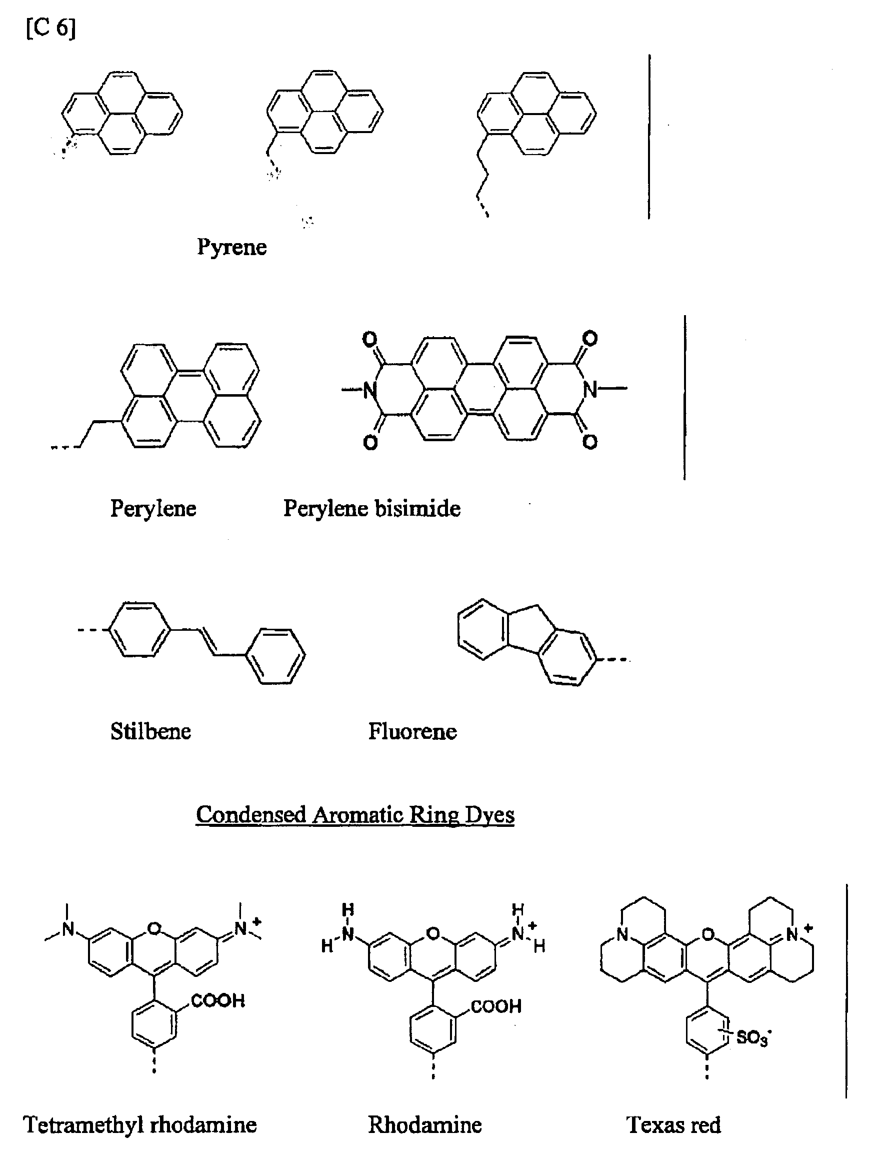

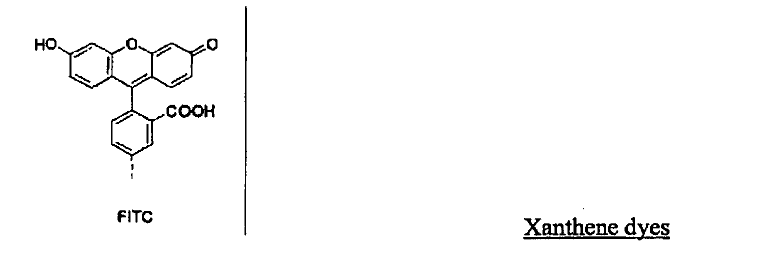

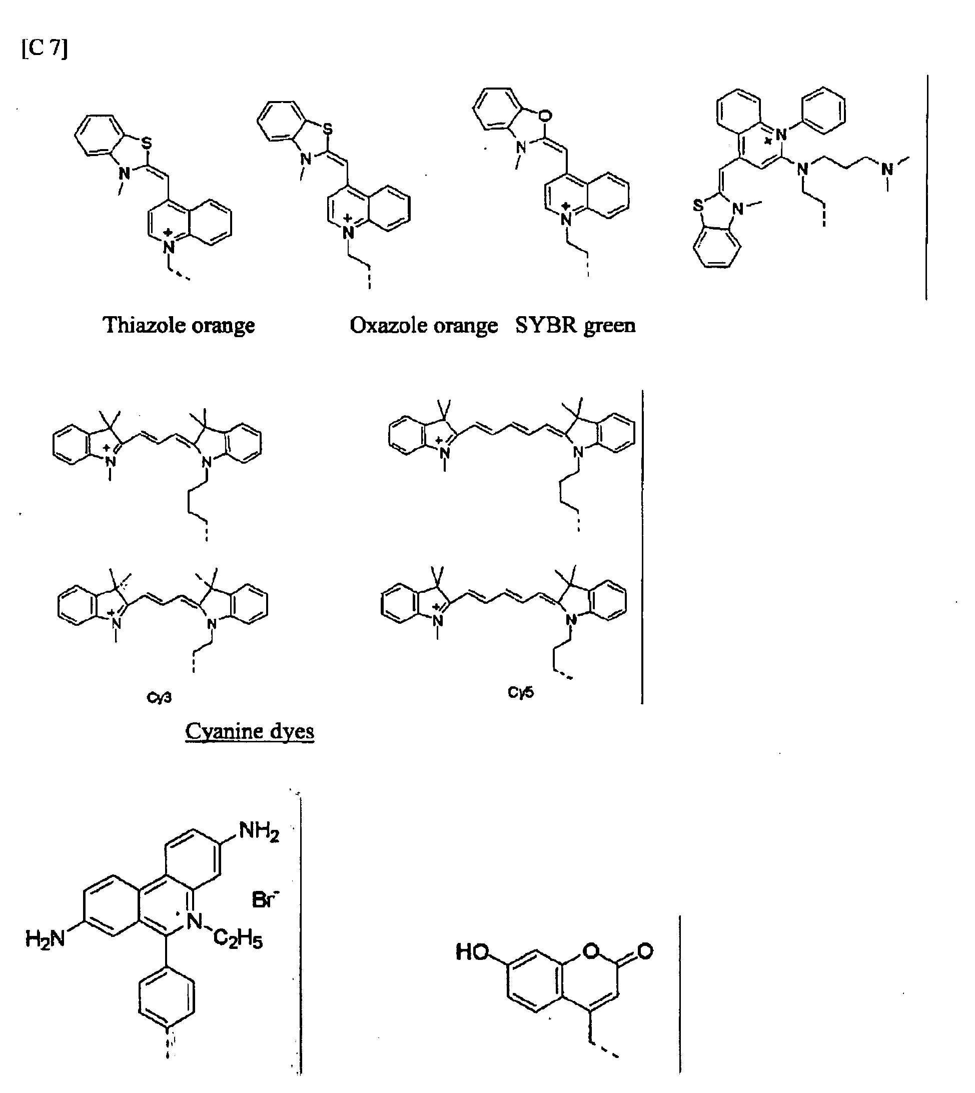

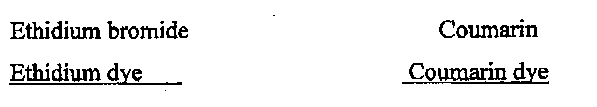

The quencher is included in the unit represented by Formula (2) below between nucleotides of the stem-forming strand. That is, quencher (Y) is provided on the stem as part of this unit or by being linked to a part of the unit other than Y. R1, R2 and Z in Formula (2) are defined as in Formula (1). The substituents in R1 and R2 are also defined as in Formula (1).

In Formula (2), Y represents the quencher. The quencher is not particularly limited as long as it is capable of quenching by forming the stack structure disclosed herein with the fluorophore used, and examples include azobenzene and derivatives thereof. All of these are photoisomerizing compounds, and their quenching action is attributed to the presence of-N=N-Any selected from methyl red, azobenzene and methylthioazobenzene is preferred as the quencher. When used with a polycyclic condensed fluorophore such as perylene, it can be said that a typical azo quencher is one for which the HOMO of the fluorophore is lower than the HOMO of the quencher, or in other words a strongly reducing quencher.

Another kind of quencher is one having an anthraquinone framework. In addition to anthraquinone, examples of compounds having anthraquinone frameworks include various anthraquinone derivatives having 1 or 2 or more functional groups selected from the hydroxyl, nitro, amino and sulfonic acid groups, halogen atoms, alkylamino groups and the like substituted for 1 or 2 or more hydrogen atoms binding to carbon atoms of the benzene ring of anthraquinone. Various anthraquinone derivatives can be easily obtained for example from Tokyo Kasei KK or the like. Examples of natural anthraquinone derivatives include cochineal dye, alizarin and other madder dyes, and lac dye compounds. Any anthraquinone derivative can be used as long as it has a LUMO lower than that of the fluorophore, but a nitro group, carboxyl group or other electron-withdrawing substituent that does not raise the LUMO energy level is preferred. Anthraquinone is most desirable. Anthraquinone and other typical anthraquinone quenchers fulfill the condition of the LUMO of the fluorophore being higher than the LUMO of the quencher when using a condensed aromatic ring fluorophore such as perylene, and can thus be called strongly oxidizing quenchers.

From the standpoint of quenching ability, the difference between the maximum absorption wavelength of the quencher and the maximum absorption wavelength of the fluorophore in a pair is preferably within 150 nm. The difference between the maximum absorption wavelength of the quencher and the maximum absorption wavelength of the fluorophore is more preferably within 100 nm, or still more preferably within 50 nm. Also, the maximum absorption wavelength of the quencher is preferably shorter than the maximum absorption wavelength of the fluorophore. The quencher can be optimized with respect to the fluorophore by controlling the maximum absorption wavelength of the quencher.

The maximum absorption wavelength of the quencher can be controlled (shifted to a longer or shorter wavelength) for example by introducing an electron withdrawing group or electron donating group into the basic framework of the quencher. For example, as shown in [C8B] below, an electron withdrawing group (such as a nitro group or halogen) or electron donating group (such as a methoxy group) can be introduced as necessary at the ortho position or para position of the azo group of the azobenzene framework of methyl red or the like.

The compound used as the quencher may be derivatized as necessary for purposes of linking it to the unit represented by Formula (2), or an optionally substituted alkylene chain may be provided between it and the linking group for purposes of pairing with the quencher. The part that links with the linking group of the quencher can be selected appropriately.

The following are examples of quenchers (Y) to be linked in Formula (2). The various compounds of the following examples are only examples, and appropriate substituents may be substituted for any of the hydrogen atoms. An optionally substituted alkylene group, alkenylene group or alkynylene group may also be interposed at the linkage (dotted line) with the linking group. Moreover, atoms on the fluorophore that is linked to a unit represented by Formula (2) are not particularly limited.

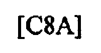

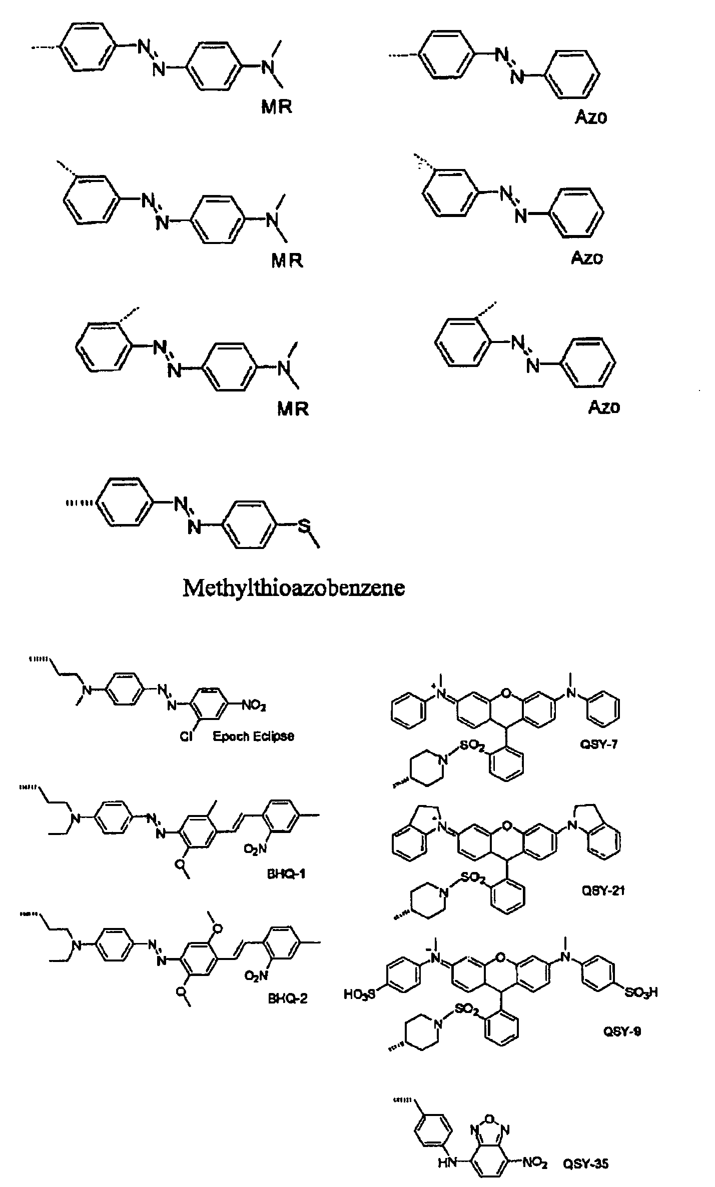

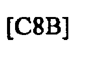

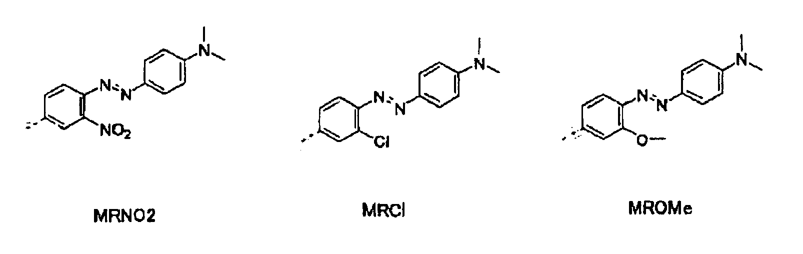

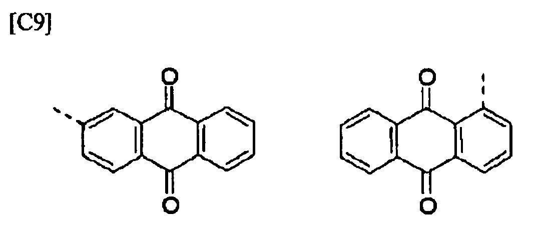

The combination of fluorophore and quencher in the disclosures of the present Specification can be selected based on the fluorescence quenching action shown below. In general there are two types of fluorescence quenching, 1) the Foerster excitation energy transfer type and 2) the electron transfer type. Fluorescence quenching based on Foerster energy transfer requires that the fluorescence spectrum of the fluorophore overlap the absorption spectrum of the quencher. Consequently, when using Foerster energy transfer, it is desirable to chose a combination of fluorophore and quencher meeting this condition. The Foerster type of quenching can occur even if there is a distance of 2 to 10 nm between the fluorophore and quencher, but since the fluorophore and quencher are in contact in the disclosures of the present Specification, Foerster type quenching is possible as long as the fluorescence-absorption spectrum condition is met.

In the 2) electron transfer type of quenching, the energy ranking relationship between the highest occupied molecular orbitals (HOMO) and lowest unoccupied molecular orbitals (LUMO) of the fluorophore and quencher is important. The fluorescence mechanism of the fluorophore is based on the release of excitation energy as fluorescence when electrons excited from HOMO to LUMO return to HOMO. When one of two electrons that are in HOMO in the fluorophore is photoexcited and moves to LUMO, the HOMO of the fluorophore is empty. When the energy ranking of the HOMO of the quencher is higher than the HOMO of the fluorophore, electrons in the HOMO of the quencher move to the emptied HOMO of the fluorophore. Since the excited fluorophore electrons can no longer return to the original HOMO, they cannot emit fluorescence. When the LUMO of the quencher is lower than the HOMO of the fluorophore, moreover, electrons that are excited into LUMO move to the LUMO of the quencher and can no longer return to the original HOMO, or emit fluorescence. Thus, in the electron transfer type of quenching a combination that fulfills the condition of either 1) the HOMO of the fluorophore being lower than the HOMO of the quencher or 2) the LUMO of the fluorophore being higher than the LUMO of the quencher is preferred. Since electron transfer only occurs at short distances, the fluorophore and quencher are preferably in contact. Since in the disclosures of the present Specification the fluorophore and quencher are in contact (stacked), adequate quenching of the electron transfer type is possible with a combination fulfilling the aforementioned conditions. Most of the combinations of fluorophore and quencher disclosed herein fulfill the conditions for causing quenching of the electron transfer type.

Quenching of a condensed aromatic ring fluorophore such as perylene by anthraquinone or its derivative occurs when excited electrons move to the LUMO of anthraquinone. This is "electron trap" quenching. In addition to the fundamental ease of electron transfer, it is believed that in electron transfer-type quenching, electron transfer is further facilitated by the strong pair formation between the anthraquinone quencher and the condensed aromatic ring fluorophore. When the number of pair formations between the anthraquinone quencher and the condensed aromatic ring fluorophore is increased, the electron transfer-type trap effect increases proportionally, and is also further enhanced because it becomes easier for electrons to move via natural base pairs from the fluorophore to a quencher not forming part of the same pair. With an azo-type quencher on the other hand, so called "hole-trap" quenching occurs, in which electrons in the HOMO of the azo-type quencher move to holes generated by excitation in the HOMO of the fluorophore. Because electrons are more mobile than holes, "electron trap" type quenching using perylene and anthraquinone is more efficient.

The fluorophore and quencher can be introduced for example using an amidite derivative for introducing such a unit instead of an amidite derivative corresponding to the nucleotides in a common solid-phase synthesis method. For example, the amino group of an aminoalkyldiol such as D-threoninol or 3-amino-1,2-propanediol is protected with a suitable allyloxycarbonyl or other protective group, while one hydroxyl group is protected with dimethoxytrityl chloride or the like, after which 2-cyanoethyl-N,N,N,N-tetraisopropyl phosphorodiamidite is introduced into the other hydroxyl group. The fluorophore or quencher can be introduced into this amidite to obtain an amidite monomer. In the case of an azobenzene compound or perylene compound for example, the methods described in Nature Protocols 2007, Vol. 2, pages 203-212, the methods described in the Journal of the American Chemical Society 2003, Vol. 125, pages 2217-2223 and the methods described in Tetrahedron Letters 2007, Vol. 48, pages 6759-6762 can be applied. When such a monomer has been obtained, an oligonucleotide provided with a unit comprising the fluorophore or quencher linked at a suitable site can by synthesized by well-known DNA synthesis methods, such as the methods described in Nature Protocols 2007, Vol. 2, pages 203-212.

The fluorophore or quencher can also be introduced after synthesis of an oligonucleotide provided at a desired location with the aforementioned amidite having an amino group protected with an allyloxycarbonyl group or the like. For example, after the amino group of an oligonucleotide provided with a unit having a protected amino group has been deprotected on a CPG carrier, a fluorophore or quencher carrying or having introduced therein a carboxylic acid group or isocyanate group capable of reacting with the amino group is reacted to thereby introduce the fluorophore or quencher.

As explained above, the oligonucleotide probe disclosed herein offers the advantages of eliminating the necessity for labeling the target nucleic acid with a fluorescent substance or the like because the probe self-generates a fluorescent signal, and allowing detection with great specificity and a high degree of flexibility in designing the target nucleic acid. The fact that the fluorophore and quencher are provided in the stem also facilitates immobilization on a solid carrier, thereby making it possible to avoid or minimize the effects of immobilization on fluorescence and quenching.

The oligonucleotide probe disclosed herein is useful not only for detecting target nucleic acids in real-time PCR and other liquid phases for which conventional molecular beacons are used, but also for analyzing patterns of gene expression, disease classifications and causative genes for diseases and the like, for gene expression monitoring including genetic diagnosis, and for detecting SNPs and other polymorphisms and various mutations. It is particularly useful for detecting polymorphisms and mutations, and for exhaustive detection of polymorphisms and mutations. In particular, because the fluorophore and quencher of the oligonucleotide probe disclosed herein are provided on the stem, the stem is more thermally stable and has a higher Tm than the same stem without a fluorophore or quencher. It can therefore have a probe sequence targeting a long sequence as the target nucleic acid, allowing for highly specific DNA analysis. In the case of an array of immobilized molecular beacons capable of simultaneously detecting a plurality of and preferably at least tens of target nucleic acids having a plurality of SNP and mutation sites, the probe sequences must be sufficiently long to distinguish the plurality of nucleic acids. This is the case with the loop of a conventional molecular beacon when the stem is lengthened, but the oligonucleotide probe disclosed herein is advantageous because the stem has a high Tm and the fluorophore can be included in the probe, thereby allowing for a longer probe sequence.

### (Immobilized probe body)

The immobilized probe body disclosed herein has a solid-phase carrier and a base sequence capable of hybridizing specifically with the aforementioned target nucleic acid, and can be provided with the oligonucleotide probe disclosed herein. In the immobilized probe body disclosed herein, a molecular beacon is preferably used as the probe.

The solid-phase carrier can be in the form of beads or a plate for example, and the material thereof is not particularly limited, but a glass or plastic solid-phase carrier can be used. Preferably the solid-phase carrier is in plate form, and is an array of 2 or more oligonucleotide probes immobilized by a particular sequence. An array is convenient for immobilizing a plurality of oligonucleotide probes and for comprehensively detecting various polymorphisms or mutations simultaneously. The array can also be provided with a plurality of individual compartmentalized array regions on a single solid-phase carrier. These individual array regions may all have oligonucleotide probes of the same set immobilized thereon, or may each have oligonucleotide probes of different sets immobilized thereon.

The mode of immobilization of the oligonucleotide probes is not particularly limited. Both covalent bonding and non-covalent bonding are possible. The oligonucleotide probes can be immobilized on the surface of the solid-phase carrier by various well-known conventional methods. Suitable linker sequences can also be provided on the surface of the solid-phase carrier. Because the oligonucleotide probe disclosed herein does not have the fluorophore and quencher at the ends of the probe, it can be easily immobilized on the solid-phase carrier by the same methods used in the past.

The uses of this immobilized probe body are not particularly limited, and it is useful for analyzing patterns of gene expression, disease classifications and causative genes for diseases and the like, for gene expression monitoring including genetic diagnosis, and for detecting SNPs and other polymorphisms and various mutations. It is particularly useful for detecting polymorphisms, mutations and the like, and for comprehensively detecting polymorphisms and mutations.

### (Method for detecting target nucleic acid)

The method for detecting a target nucleic acid disclosed herein may be a method using the oligonucleotide probe disclosed herein, which is an oligonucleotide probe having a base sequence capable of hybridizing specifically with a target nucleic acid, and detecting a signal based on the fluorophore of the molecular beacon. In the detection method disclosed herein, the oligonucleotide probe is preferably a molecular beacon, and it is more desirable to use an immobilized body of a plurality of oligonucleotide probes (typically molecular beacons) immobilized on a solid-phase carrier.

The detection method disclosed herein can be implemented for example as follows. First, a plurality of molecular beacons capable of hybridizing specifically with a plurality of target nuclear acids are immobilized on a solid-phase carrier to prepare an array. Next, a nucleic acid sample (typically obtained by extracting nucleic acids from a biological sample collected from blood or tissue, or by amplifying such a sample by PCR or the like under specific conditions) is supplied to the molecular beacons on the array in the presence of a hybridization liquid of a specific composition. A hybridization reaction is performed under specific hybridization conditions. The hybridization temperature is preferably a temperature lower than either the Tm of the stem or the Tm of the probe sequence with the target nucleic acid. After passage of a predetermined time, the array is washed, and hybridization with a target nucleic acid in the nucleic acid sample is detected by detecting the fluorescent signal emitted by the fluorophore. The fluorescent signal can be detected with a fluorescence scanner or the like. The nucleic acid is detected, identified, assayed and otherwise analyzed as necessary based on the resulting fluorescent signal.

The disclosures of the present Specification are explained in detail below using examples, but the disclosures of the present Specification are not limited by these examples.

### [Example 1]

### Amidite monomerization of D-threoninol protected with (allyloxy)carbonyl group

In this example, the amidite monomer (Compound A) of a unit (C₃) for linking the fluorophore or quencher was synthesized according to the following scheme. D-threoninol (0.99 g, 9.41 mmol) was dissolved in 75 ml of tetrahydrofuran (THF) in a 300 ml eggplant flask, and agitated after addition of 15 ml of triethylamine. Next, allyl chloroformate (1.01 ml, 9.51 mmol) previously dissolved in 75 ml of THF was dripped into the aforementioned THF solution in an ice bath. After 15 minutes the ice bath was removed and this was warmed to room temperature, agitation was continued, and the reaction was terminated 1 hour and 30 minutes after completion of THF solution dripping. The solvent was distilled away with an evaporator, and the remainder was purified by silica gel column chromatography (developing solvent chloroform:methanol = 3:1) to obtain Compound 1-1.

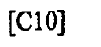

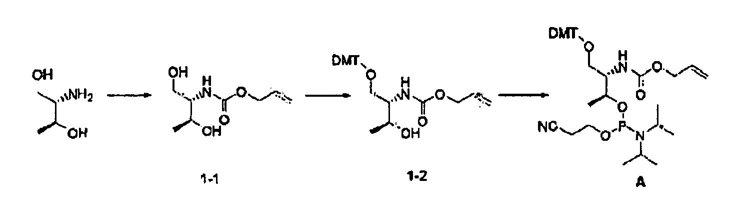

The resulting Compound 1 (1.72 g, 9.09 mmol) was collected in a 200 ml double-mouthed eggplant flask, dissolved by addition of 30 ml of anhydrous pyridine in nitrogen, and agitated after addition of N,N,diisopropylethylamine (DIPEA: 1.54 mL, 9.09 mmol). Dimethoxytrityl chloride (DMT-Cl: 3.08 g, 9.09 mmol) and dimethylaminopyridine (DMAP: 0.14 g, 1.14 mmol) were added to a 50 ml double-mouthed eggplant flask, and dissolved by addition of 10 ml of anhydrous dichloromethane as a solvent. This dichloromethane solution was then dripped slowly into the previous pyridine solution in an ice bath. After about 15 minutes of agitation with ice cooling, the ice bath was removed and agitation was continued at room temperature, and the reaction was terminated 4 hours and 30 minutes after the dichloromethane solution dripping. The solvent was distilled away with an evaporator, and the remainder was purified by silica gel column chromatography (developing solvent hexane:ethyl acetate:triethylamine = 66:33:3) to obtain Compound 1-2.

Compound 1-2 (0.74 g, 1.51 mmol) was collected in a double-mouthed eggplant flask, azeotropically dehydrated three times with 8 ml of anhydrous acetonitrile, dissolved by addition of 30 ml of anhydrous acetonitrile, and then agitated after addition of 2-cyanoethyl N,N,N',N'-tetraisopropylphosphordiamidite (0.54 g, 1.79 mmol). 1H-tetrazole (0.137 g, 1.51 mmol) was collected in a separate double-mouthed eggplant flask, azeotropically dehydrated three times with 8 ml of anhydrous acetonitrile, and dissolved by addition of 15 ml of anhydrous acetonitrile. This 1H-tetrazole solution was dripped into the previous acetonitrile solution of Compound 1-2 with ice cooling, the ice bath was removed after about 15 minutes of agitation, the solution was returned to room temperature and agitation was continued. The reaction was terminated after about 1 hour and 30 minutes. The solvent was distilled away with an evaporator, and the remaining oily compound was dissolved by addition of ethyl acetate. This ethyl acetate solution was shaken twice with a saturated aqueous solution of sodium bicarbonate using a separating funnel, and then shaken twice more in the same way with a saturated aqueous solution of sodium chloride. Magnesium sulfate was then added to remove the moisture, the ethyl acetate was distilled away with an evaporator, and the remainder was purified by silica gel column chromatography (developing solvent hexane:ethyl acetate:triethylamine = 50:50:3) to obtain Compound A.

### [Example 2]

### Amidite monomerization of 3-amino-1,2-propanediol protected with (allyloxy)carbonyl group

In this example, the amidite monomer (Compound B) of a unit (C₂) for linking the fluorophore or quencher was synthesized by the following scheme.

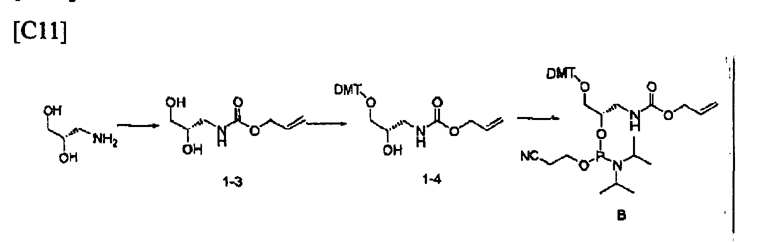

3-amino-1,2-propanediol (1.0 g, 11 mmol) was dissolved in 30 ml of dimethylformamide (DMF) in a 200 ml eggplant flask, and agitated after addition of 15 ml of triethylamine. Allyl chloroformate (1.4 ml, 11 mmol) previously dissolved in 20 ml of DMF was then dripped in to the previous DMF solution in an ice bath. 15 minutes later the ice bath was removed, the solution was returned to room temperature, agitation was continued, and the reaction was terminated 2 hours after the completion of dripping of the DMF solution. The solvent was then distilled away with an evaporator, and the remainder was purified by silica gel column chromatography (developing solvent chloroform:methanol = 3:1) to obtain Compound 1-3. Compound 1-4 and then Compound B were obtained by methods similar to those of Example 1, but using Compound 1-3 as the raw material.

The Compound C shown below was obtained by the methods described in Nature Protocols 2007, Vol. 2, pp. 203-212, while Compound D was obtained by the methods described in the Journal of the American Chemical Society 2003, Vol. 125, pp. 2217-2223, and Compound E was obtained by the methods described in Tetrahedron Letters 2007, Vol. 48, pp. 6759-6762.

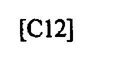

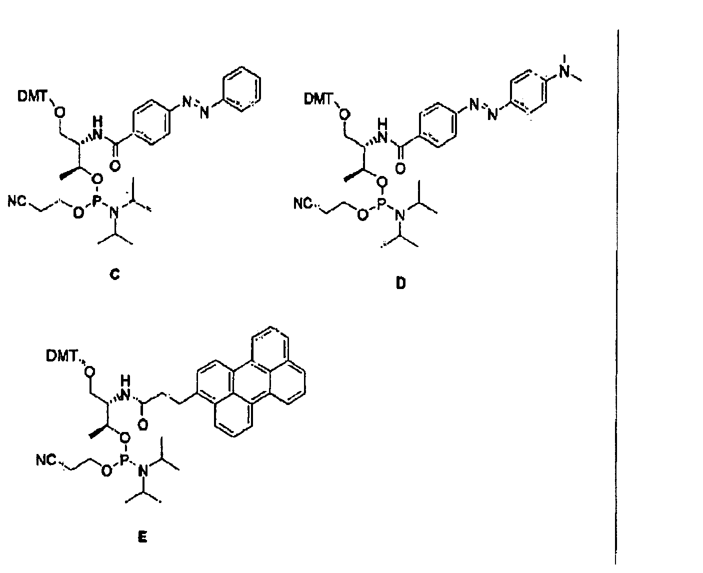

### [Example 3]

### Synthesis of thiazole orange (TO-1, TO-2)

In this example, thiazole orange derivatives were synthesized according to the following scheme as fluorophores.

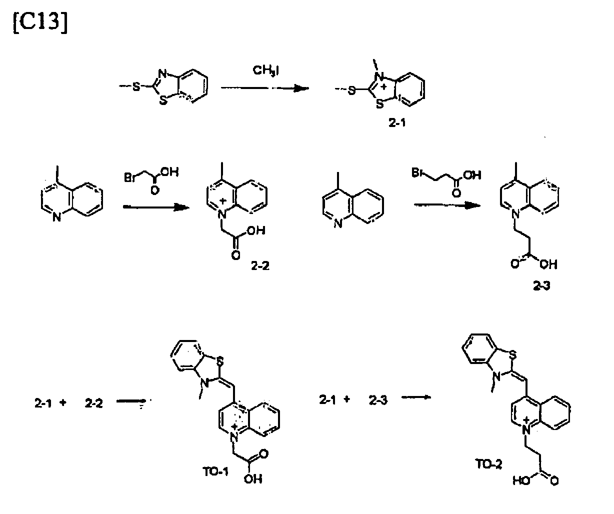

2-(methylthio)benzothiazole (2.04 g, 11.3 mmol) was measured into a 200 ml eggplant flask and dissolved by addition of 5 ml of ethanol, and this solution was heated to reflux at 65°C for 3 hours after addition of iodomethane (1.5 ml, 24.1 mmol). The resulting precipitate was collected by suction filtration to obtain Compound 2-1 (0.52 g).

Bromoacetic acid (1.94 g, 14.0 mmol) was then measured into a 200 ml eggplant flask, and dissolved by addition of 10 mL ethyl acetate. Lepidine (2 ml, 15.1 mmol) was then added, and agitated for 2 hours 30 minutes at room temperature. The resulting precipitate was collected by suction filtration to obtain Compound 2-2 (0.41 g). Bromobutyric acid (8.08 g, 52.8 mmol) was then measured into a 200 ml eggplant flask as above, and dissolved by addition of 10 ml ethyl acetate. Lepidine (7 ml, 52.8 mmol) was then added, and agitated overnight at 80°C. The resulting precipitate was then collected by suction filtration to obtain Compound 2-3 (4.85 g).

For the thiazole orange TO-1, Compound 2-1 (0.42 g, 2 mmol) and Compound 2-2 (0.41 g, 2 mmol) were dissolved by addition of 10 ml anhydrous ethanol and refluxed for 5 minutes at 60°C, triethylamine (0.27 ml, 2 mmol) was added, and once the mixture turned red it was returned to room temperature with agitation. A precipitate obtained by adding 150 ml of diethyl ether to this red reaction mixture was collected by suction filtration to obtain thiazole orange (TO-1, 0.59 g). Thiazole orange TO-2 was obtained by the same methods using Compound 2-3.

### [Example 4]

### Oligonucleotide synthesis

In this example, the following oligonucleotides with introduced quenchers and oligonucleotides with introduced fluorophores were synthesized. PE represents perylene, MR represents methyl red and Azo represents azobenzene.

**[Table 1]**

| Type of oligonucleotide | Sequence | R1 | X or Y |
|---|---|---|---|
| Fa-C3-TO1 | 5'-GGTATC-R1-GCAATC-3' | C3 | TO1 |
| Fa-C3-TO2 | 5'-GGTATC-R1-GCAATC-3' | C3 | TO2 |
| Fa-C3-FITC | 5'-GGTATC-R1-GCAATC-3' | C3 | FITC |
| Fa-C3-PE | 5'-GGTATC-R1-GCAATC-3' | C3 | PE |
| Fa-C2-TO1 | 5'-GGTATC-R1-GCAATC-3' | C2 | TO1 |
| Fa-C2-TO2 | 5'-GGTATC-R1-GCAATC-3' | C2 | TO2 |
| Fa-C2-FITC | 5'-GGTATC-R1-GCAATC-3' | C2 | FITC |
| Qb-MR | 3'-CCATAG-R1-CCTTAG-5' | C3 | MR |
| QB-Azo | 3'-CCATAG-R1-CCTTAG-5' | C3 | Azo |

Synthesis of oligonucleotides with introduced azobenzene, methyl red and perylene
DNA with azobenzene, methyl red or perylene introduced as the quencher was synthesized and purified by the methods described in Nature Protocols 2007, Vol. 2, pp. 203-212 with an ABI394 DNA synthesizer using the corresponding phosphoroamidite monomers (Compounds C, D and E) and commercial phosphoroamidite monomers corresponding to four natural nucleotides.

Introduction of thiazole orange (TO-1, TO-2) and FITC into oligonucleotides
Introduction ofthiazole orange (TO-1, TO-2) and FITC into oligonucleotides was performed by the methods shown in the following scheme via phosphoroamidite monomers protected with allyloxycarbonyl groups.

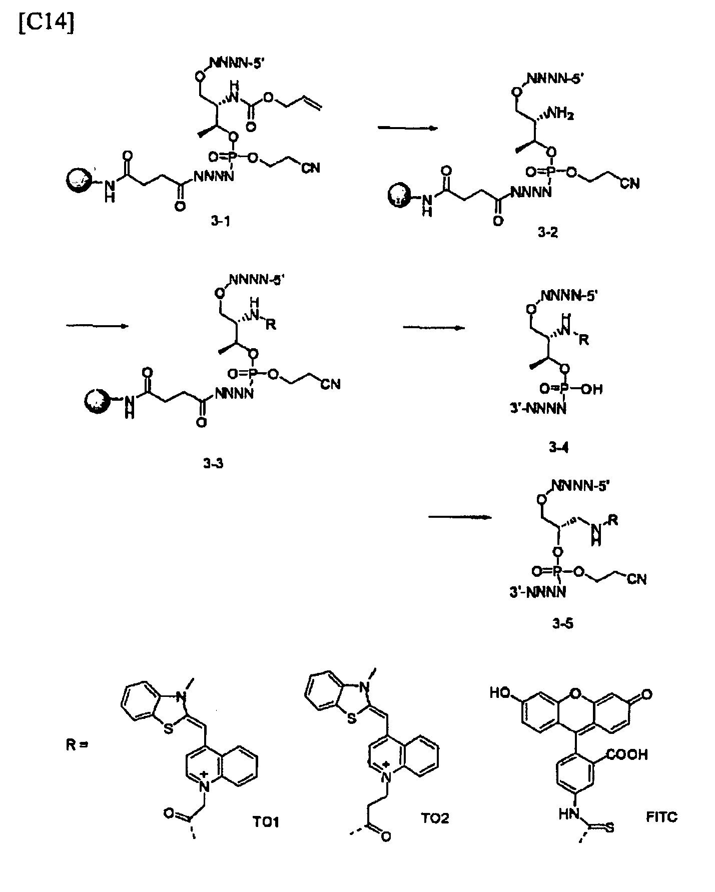

Using an ABI394 DNA synthesizer, DNA having a specific sequence was elongated on a controlled pore glass (CPG) carrier using phosphoroamidite monomer A having the amino group of D-threoninol protected with an allyloxycarbonyl group, suitable amounts of four different commercial phosphoroamidite monomers corresponding to natural nucleotides, and the amidite monomers C, D and E described above (Compound 3-1). This CPG carrier (10 mg, 0.45 µmol) was measured into a plastic syringe equipped with a filter, and washed three times with 1 mL of acetonitrile and three times with 1 mL of dichloromethane. 48.8 µL (450 µmol) of N-methylaniline was then added to 500 µL of a dichloromethane solution of dissolved Pd(Ph₃)₄ (5.2 mg, 4.5 µmol), and this was added to the aforementioned CPG carrier (Compound 4-1) and reacted for 3 hours at 35°C to deprotect only the allyloxycarbonyl groups from the CPG carrier (Compound 3-2).

Thiazole orange TO-1 (10 mg, 45 µmol) and pyridinium para-toluenesulfonate (PPTS: 11.3 mg, 45 µmol) were then dissolved in 400 µL ofDMF, 100 µl of a DMF solution of benzotriazole-1-yl-oxytripyrrolidinophosphonium hexafluorophosphate (PyBOP: 59 mg, 112 µmol) was added, and the mixture was added to a syringe containing the aforementioned CPG carrier (Compound 3-2) and shaken for 3 days at room temperature. The reaction solvent was removed by filtration, and 1 mL of a DMF solution of 0.1 M PPTS was added to the syringe and shaken for 1 minute to wash the CPG carrier, which was then washed three times with 1 mL DMF and three times with 1 ml dichloromethane to obtain a CPG carrier with introduced TO-1 (Compound 3-3).

DNA was then excised from the CPG carrier by the methods described in Nature Protocols 2007, Vol. 2, pp. 203-212, and the target DNA was separated and purified by the methods described in the same article using high-speed liquid chromatography (Compound 3-4).

DNA with introduced thiazole orange TO-2 was obtained by the same methods, but using TO-2 in place of TO-1.

DNA with introduced FITC was prepared by adding DIPEA (6.12 µl, 18 µmol) to a DMF solution (500 µL) of FITC (14.02 mg,18 µmol), adding this to a CPG carrier (10 mg, 0.36 µmol) (Compound 3-2) having only the allyloxycarbonyl groups deprotected, shaking the mixture for 3 days and then carrying out the same process as in the case of DNA with introduced TO.

An oligonucleotide comprising linker R1 (C₂) was synthesized by introducing TO-1, TO-2 or FITC by exactly the same methods, but using Compound B in place of Compound A (Compound 3-5).

### [Example 5]

Confirming fluorescence quenching caused by combination with the quencher
To confirm quenching by "base pair formation" between various fluorophores and quenchers, the fluorescence and absorption spectra were measured using the oligonucleotides synthesized in Example 4. The solution conditions for absorption spectrum and fluorescence spectrum measurement were as follows.

| | |
|---|---|
| Fa sequence oligonucleotide: | 5 µmol/l |
| Qb sequence or Nb oligonucleotide: | 5 µmol/l |
| Sodium chloride: | 0.1 mol/l |
| Phosphate buffer: | 10 mmol/l (pH 7.0) |

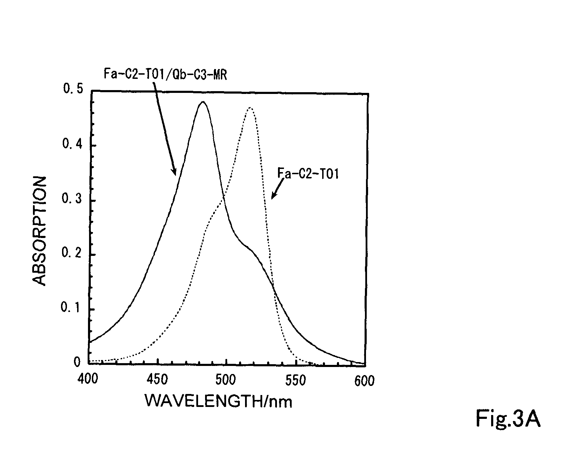

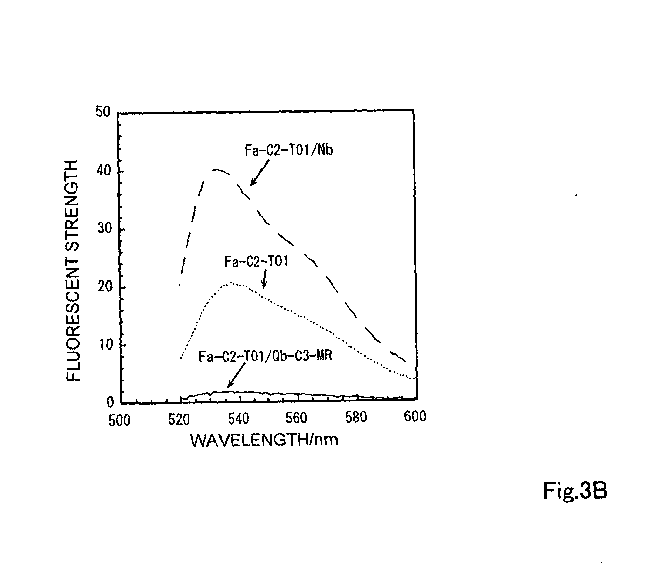

Oligonucleotides (without quenchers) (Nb) having sequences complementary to Fa were prepared separately as the target nucleic acids for the various oligonucleotides Fa including fluorophores. The results are shown in Figs. 3A and 3B.

Fig. 3A shows the absorption spectra at 20°C of Fa-C2-TO1 (single strand) and a Fa-C3-TO1/Qb-MR double strand, respectively. Fig. 3B shows the fluorescence spectra. As shown in Fig. 3A, 515 nm absorption based on thiazole orange diminished greatly due to double strand formation with Qb-MR. This is evidence of stacking between the methyl red quencher and the thiazole orange. Moreover, the melting temperature Tm of the Fa-C2-TO 1/Qb-MR double strand was 54°C, higher than the Tm (48°C) of the natural DNA double strand (5'-GGTATCGCAATC-3'/3'-CCATAGCGTTAG-5') without thiazole orange or methyl red. This is also evidence of strong stacking between the thiazole orange and methyl red. Similar absorption spectrum changes and elevated Tm values were observed with Fa-C2-TO2/Qb-MR, Fa-C3-TO1/Qb-MR and Fa-C3-TO2-Qb-MR.

In the fluorescence spectra shown in Fig. 3B (511 nm excitation), the fluorescent strength at 533 nm based on thiazole orange from the Fa-C2-TO1/Qb-MR double strand was 1/10 that of the Fa-C2-TO1 in a single strand state. When the Fa-C2-TO1 was made to form a double strand with a complementary oligonucleotide (Nb), however, the fluorescent strength at 533 nm was increased to twice that of the Fa-C2-TO1 single strand at 20°C. This shows that an about 20-fold increase in the signal/noise ratio can be expected when a beacon is prepared with this combination.

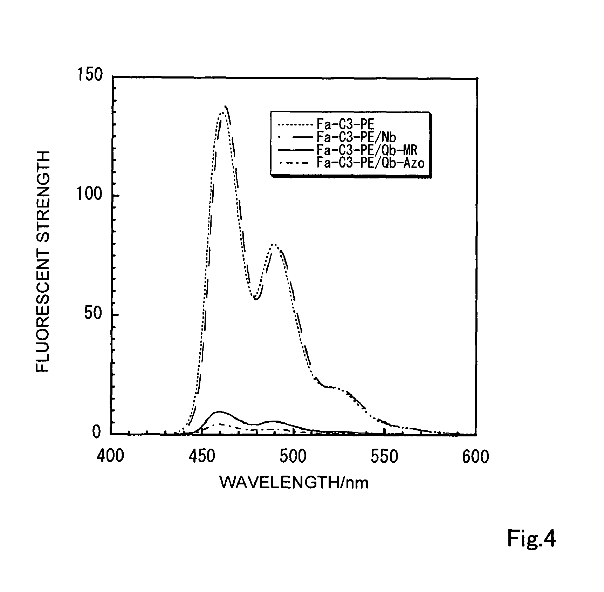

Next, double strands were prepared with Qb-MR or Qb-Azo and Fa-C3-PE containing perylene. The results are shown in Fig. 4. As shown in Fig. 4, the fluorescent strength at 461 nm after excitation at 425 nm was reduced to 1/15 with Qb-MR and 1/30 with Qb-Azo at 20°C in comparison with the single-strand Fa-C3-PE as shown in Fig. 4. When the Fa-C3-PE was made to form a double strand with Nb, on the other hand, the resulting fluorescence was similar to that of the single-strand Fa-C3-PE. This shows that a roughly 30-fold increase in the signal/noise ratio at about 20°C can be expected from a combination of azobenzene with perylene.

### [Example 6]

Based on the results of Example 5, the molecular beacon-type oligonucleotide probes shown in Table 2 below were designed and synthesized. The various molecular beacons were synthesized according to the methods described in Example 4 using the various amidite monomers, fluorophores and quenchers prepared in Examples 1 to 3. The respective sequences are also shown below.

**[Table 2]**

| Beacon type | Sequence | R1 | X | R1 | Y |
|---|---|---|---|---|---|
| MB1-C2-TO1-MR | MB1 | C2 | TO1 | C3 | MR |
| MB2-C2-TO1-MR | MB2 | C2 | TO1 | C3 | MR |
| MB1-C2-FITC-MR | MB1 | C2 | FITC | C3 | MR |
| MB2-C2-FITC-MR | MB2 | C2 | FITC | C3 | MR |
| MB1-C3-PE-Azo | MB1 | C3 | PE | C3 | Azo |
| MB2-C3-PE-Azo | MB2 | C3 | PE | C3 | Azo |
| MB-t | MBt | | | | |

### [C15]

| | |
|---|---|
| MB1: | 5'-TG-X-GTCCTTGAG-AAAGGGCGAC-Y-CA-3' |
| MB2: | 5'-TGG-X-TCCTTGAG-AAAGGGCGA-Y-CCA-3' |
| MB0F: | 5'-FITC-TGGTCCTTGAG-AAAGGGCGACCA-MR-3' |
| MBt: | 3'-ACCAGGAACTCTTTCCCG-5' |

| | |
|---|---|
| *underline indicates a stem-forming strand. | |

The 5' terminal FITC and 3' terminal MR used in the MB0F comparative example had the following structures, differing from the linkers used for introduction into the beacons disclosed herein.

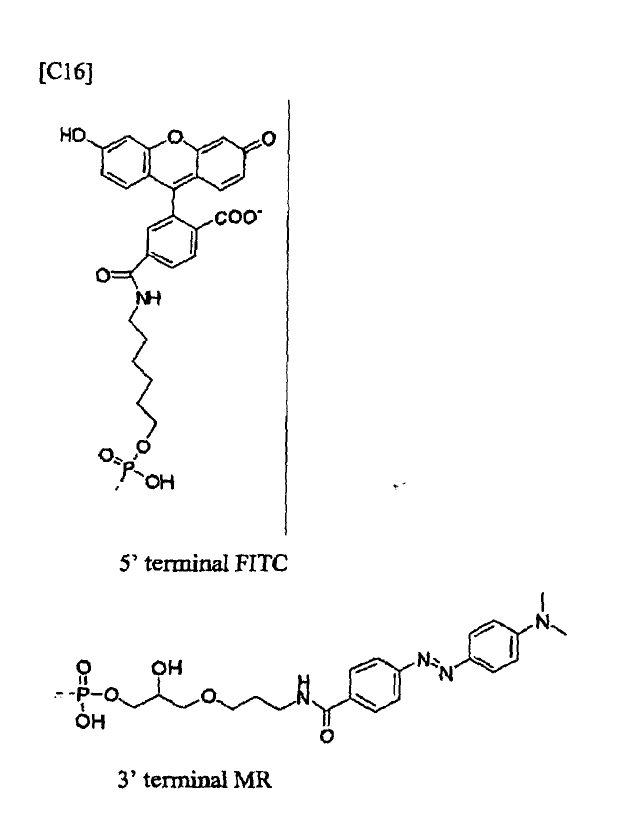

Changes in fluorescent strength were observed before and after addition of the target nucleic acid MBt to the beacons MB1-C2-TO1-MR and MB2-C2-TO1-MR. The solution conditions for fluorescence spectrum measurement were as follows. The results are shown in Tables 3 and 4.

| | |
|---|---|
| Beacon concentration: | 0.2 µmol/l |
| Target nucleic acid Nb concentration: | 0.4 µmol/l |
| Sodium chloride: | 0.1mol/l |
| Phosphate buffer: | 10 mmol/l (pH 7.0) |

### MB1-C2-TO1-MR

The following table shows the fluorescent strength at 531 nm from thiazole orange after excitation at 520 nm before and after addition of MB-t to MB1-C2-TO1-MR.

**[Table 3]**

| °C | Before MB-t addition | After MB-t addition | Fluorescent strength change before and after MB-t addition: Signal strength ratio |
|---|---|---|---|
| 0 | 80 | 887 | 11.1 |
| 10 | 63.5 | 673 | 10.1 |
| 20 | 45.3 | 483 | 10.7 |
| 30 | 34.4 | 337 | 9.9 |
| 37 | 24.9 | 253 | 10.2 |
| 40 | 23.2 | 217 | 9.4 |
| 50 | 15.8 | 131 | 8.3 |
| 60 | 13.1 | 45 | 3.4 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | High |
| Scanning speed: | 200 nm/min |

As shown in Table 3, the background value (before MB-t addition) of this molecular beacon-type probe is roughly stable across a broad temperature range, and the signal strength ratio is also stabilized across a broad temperature range. In comparison with the comparative example shown below, this molecular beacon-type probe is shown to exhibit excellent thermal stability of the stem. Because the signal strength ratio is stabilized, moreover, it is shown to be useful for detecting SNPs, mutations and other gene variants.

### MB2-C2-TO1-MR

The fluorescent strength at 531 nm from thiazole orange excited at 510 nm before and after addition of MBt to MB2-C2-TO1-MR is shown below.

**[Table 4]**

| °C | Before MB-t addition | After MB-t addition | Fluorescent strength change before and after MB-t addition: Signal strength ratio |
|---|---|---|---|
| 10 | 21.2 | 527 | 24.8 |
| 20 | 18 | 376 | 20.9 |
| 30 | 15.2 | 267 | 17.6 |
| 37 | 13.5 | 193 | 14.3 |
| 40 | 12.8 | 170 | 13.3 |
| 50 | 11.4 | 107 | 9.4 |
| 60 | 9.3 | 35.7 | 3.8 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | High |
| Scanning speed: | 200 nm/min |

As shown in Table 4, the background (before MB-t addition) of this molecular beacon-type probe was roughly stable between 30°C and 50°C, and the signal strength ratio also tended to stabilize within this range. In comparison with the comparative example shown below, this molecular beacon-type probe exhibited a tendency toward superior thermal stability of the stem. Because the signal strength ratio is stabilized, moreover, it is shown to be useful for detecting SNPs, mutations and other variants.

### Comparative example: MB0F

As a comparative example, fluorescent strength at 520 nm from FITC excited at 494 nm is shown below before and after addition of MBt to MB-cont

**[Table 5]**

| °C | Before MB-t addition | After MB-t addition | Fluorescent strength change before and after MB-t addition: Signal strength ratio |
|---|---|---|---|
| 0 | 1.3 | 281 | 216 |
| 10 | 2.1 | 282 | 134 |
| 20 | 2.5 | 283 | 113 |
| 30 | 3.8 | 280 | 74 |
| 37 | 7.4 | 280 | 37 |
| 40 | 10 | 281 | 28 |
| 50 | 39 | 270 | 6.9 |
| 60 | 93 | 188 | 2 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Medium |
| Scanning speed: | 200 nm/min |

As shown in Table 5, the background of a conventional molecular beacon (before MB-t addition) increased as the temperature increased, increasing rapidly at 40°C and above. Emission (after MB-t addition) due to hybridization decreased dramatically at 50°C and more. As a result, the signal strength ratio declined rapidly at 40°C and above. This is unsuitable for detecting SNPs, mutations and the like since relatively high hybridization temperatures are considered necessary for detecting SNPs and mutations. It is also shown to be unsuitable for solid-phase reactions, since even greater variation in the solid phase can be anticipated from this instability.

### MB1-C3-PE-Azo

Fluorescent strength at 460 nm from perylene excited at 425 nm is shown below before and after addition of MBt to MB1-C3-PE-Azo.

**[Table 6]**

| C | Before MB-t addition | After MB-t addition | Fluorescent strength change before and after MB-t addition: Signal strength ratio |
|---|---|---|---|
| 0 | 4.4 | 78.3 | 17.8 |
| 10 | 4.8 | 73.6 | 15.3 |
| 20 | 5.4 | 67.3 | 12.5 |
| 30 | 6.3 | 60.5 | 9.6 |
| 40 | 7.9 | 53.1 | 6.7 |
| 50 | 11 | 45.1 | 4.1 |
| 60 | 17.3 | 30.9 | 1.8 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

### MB2-C3-PE-Azo

Fluorescent strength at 460 nm from perylene excited at 425 nm is shown below before and after addition of MBt to MB2-C3-PE-Azo.

**[Table 7]**

| °C | Before MB-t addition | After MB-t addition | Fluorescent strength change before and after MB-t addition: Signal strength ratio |
|---|---|---|---|
| 0 | 26 | 93.3 | 3.6 |
| 10 | 24.3 | 89.1 | 3.7 |
| 20 | 23.5 | 83.7 | 3.6 |
| 30 | 22.8 | 77.8 | 3.4 |
| 40 | 23.4 | 71.1 | 3 |
| 50 | 25.5 | 62.1 | 2.4 |
| 60 | 28.4 | 41.7 | 1.5 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

As shown in Tables 6 and 7, changes in the fluorescent strength ratio due to differences in hybridization temperature were controlled.

### [Example 7]

The compound Anth shown below was obtained by the methods described in Chemical Communications 2006, Vol. 5062-5064.

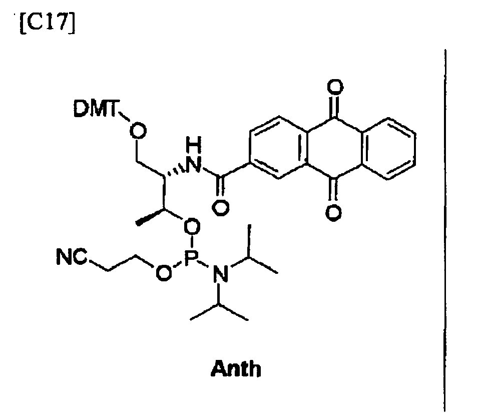

### Synthesis of amidite monomer containing 4-[4-methylthiophenylazo]benzoic acid

An amidite monomer containing 4-[4-methylthiophenylazo]benzoic acid was synthesized according to the following scheme. First, ethyl para-nitrosobenzoate (4-1) was synthesized by the methods described in the Journal of the Organic Chemistry 1970, Vol. 35, pp. 505-508. This nitroso compound 4-1 was placed in a double-mouthed eggplant flask, nitrogen was substituted, p-methylthioaniline 4-2 was added, acetic acid was added as a solvent, and the mixture was reacted overnight with light shielding. Distilled water was added to this reaction solution, followed by ethyl acetate, and the mixture was shaken thoroughly with a separating funnel. The ethyl acetate layer was washed successively with distilled water, saturated sodium bicarbonate aqueous solution and saturated sodium chloride aqueous solution and dried with magnesium sulfate, and the ethyl acetate solvent was distilled away. The product was then further purified by silica gel column chromatography (hexane:ethyl acetate =15:1) to obtain Compound 4-3 (yield 50%).

Ethanol and 2 N sodium hydroxide aqueous solution were added to dissolve the resulting Compound 4-3, which was then agitated for 17 hours with light shielding. Hydrochloric acid was then added to a pH of about 5, ethyl acetate was added, and the mixture was shaken thoroughly with a separating funnel. Next, the ethyl acetate layer was washed successively with distilled water, saturated sodium bicarbonate aqueous solution and saturated sodium chloride aqueous solution and dried with magnesium sulfate, and the ethyl acetate solvent was distilled away to obtain para-nitrosobenzoic acid 4-4 (yield 99% or more). Next, amidite monomer 4-5 was obtained according to the methods described in Nature Protocols 2007, Vol. 2, pp. 203-212, except that para-nitrosobenzoic acid 4-4 was used instead of azobenzene.

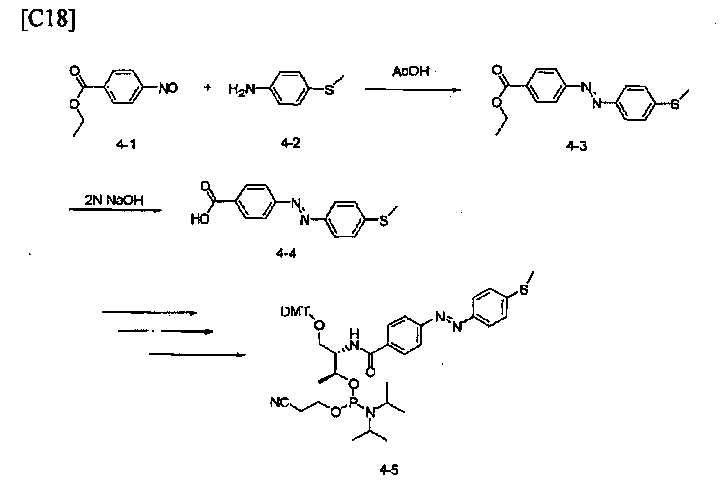

### Synthesis of amidite monomer containing Cy3

An amidite monomer containing Cy3 was synthesized according to the following scheme. First, Cy3 (5-1) was obtained by the methods described in the Journal of the American Chemical Society 2001, Vol. 125, pp. 361-362. The amidite monomer 5-2 was then obtained according to the methods described in Nature Protocols 2007, Vol. 2, pp. 203-212, except that Cy3 (5-1) was used instead of azobenzene.

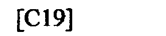

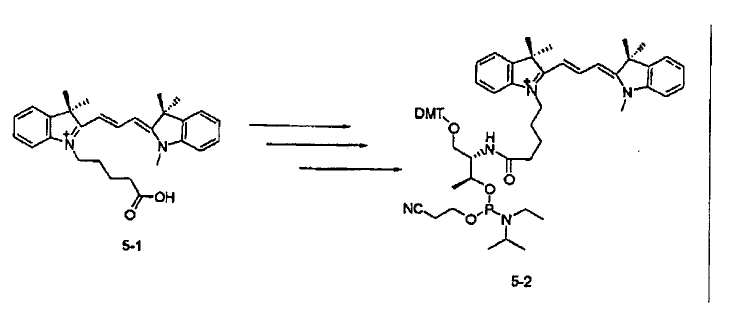

The molecular beacon-type oligonucleotide probes shown in Table 8 below were then designed and synthesized. Each molecular beacon was synthesized in accordance with the methods described in Example 4 using the various amidite monomers, fluorophores and quenchers synthesized or provided in Examples 1 to 3. The various sequences are also shown.

**[Table 8]**

| Beacontype | Sequence | X | R | Y | R |
|---|---|---|---|---|---|
| MBt | MBt | | | | |
| MB1-C3-PE-S | MB1 | C3 | PE | C3 | S |
| MB2-C3-PE-S | MB2 | C3 | PE | C3 | S |
| MB1-C3-PE-Q | MB1 | C3 | PE | C3 | Q |
| MB2-C3-PE-Q | MB2 | C3 | PE | C3 | Q |
| MB3-C3-PE-Q | MB3 | C3 | PE | C3 | Q |
| MB1-C3-Cy3-MR | MB1 | C3 | Cy3 | C3 | MR |
| MB2-C3-Cy3-MR | MB2 | C3 | Cy3 | C3 | MR |

The abbreviations in Table 8 are the same as those used in the following figures. The abbreviations used in Table 2 of Example 6 are also the same as those used in the following figures.

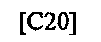

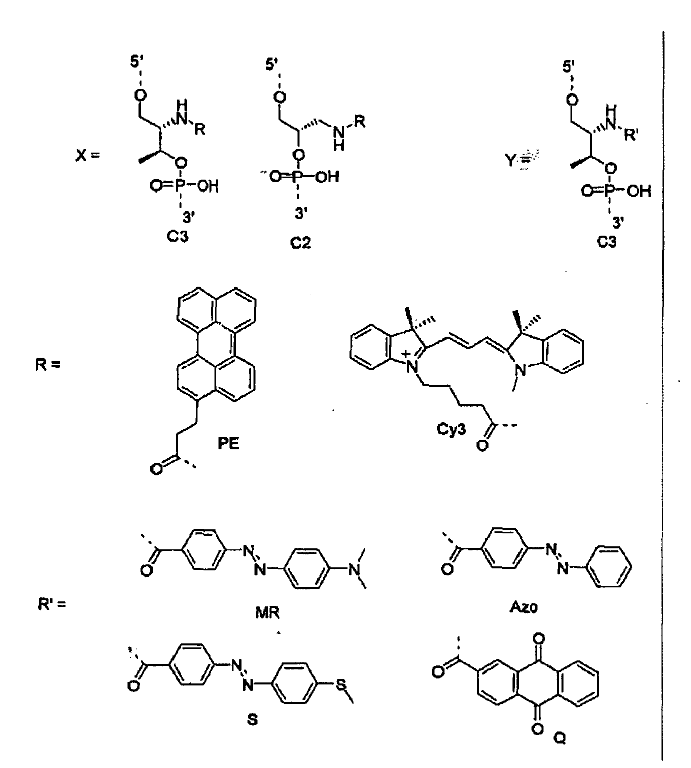

| | |
|---|---|
| MBt: | 5'-TG-X-GTCCTTGAG-AAAGGGCGAC-Y-CA-3' |
| MB2: | 5'-TGG-X-TCCTTGAG-AAAGGGCGA-Y-CCA-3' |
| MB3: | 5'-T-X-GG-X-TCCTTGAG-AAAGGGCGA-Y-CC-Y-A-3' |
| MBt: | 3'-ACCAGGAACTCTTTCCCG-5' |

Changes in the fluorescent strength of these beacons were observed before and after addition of the target nucleic acid MBt. The solution conditions for fluorescence spectrum measurement were as follows. The results are shown in Tables 9 to 15.

| | |
|---|---|
| Beacon concentration: | 0.2 µmol/l |
| Target nucleic acid Nb concentration: | 0.4 µmol/l |
| Sodium chloride: | 0.1 mol/l |
| Phosphate buffer: | 10 mmol/l (pH 7.0) |

1. Confirmation of quenching-emission from perylene-(4-[4-methylthiophenylazo]benzoic acid
MB1-C3-PE-S
The following table shows the fluorescent strength at 460 nm from perylene excited at 425 nm before and after addition of MBt to MB1-C3-PE-S.

**[Table 9]**

| °C | Before MB-t addition | After MB-t addition | Fluorescent strength change before and after MB-t addition: Signal strength ratio |
|---|---|---|---|
| 0 | 5.2 | 84.7 | 16.3 |
| 10 | 5.1 | 79.2 | 15.5 |
| 20 | 5.1 | 75.8 | 12.5 |
| 30 | 5.3 | 68.7 | 13 |
| 40 | 5.7 | 60.3 | 10.6 |
| 50 | 7.2 | 51.2 | 7.1 |
| 60 | 11.6 | 32.9 | 2.8 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

### MB2-C3-PE-S

The following table shows the fluorescent strength at 460 nm from perylene excited at 425 nm before and after addition of MBt to MB2-C3-PE-S.

**[Table 10]**

| °C | Before MB-t addition | After MB-t addition | Fluorescent strength change before and after MB-t addition: Signal strength ratio |
|---|---|---|---|
| 0 | 3.1 | 48.5 | 15.6 |
| 10 | 3 | 46.2 | 15.7 |
| 20 | 2.9 | 43.8 | 15.1 |
| 30 | 3.2 | 41 | 13 |
| 40 | 3.9 | 37.8 | 9.7 |
| 50 | 5.8 | 32.4 | 5.6 |
| 60 | 9.1 | 15.8 | 1.7 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

As shown in Tables 9 and 10, quenching-emission behavior attributable to supplying of the target nucleic acid was confirmed using 4-[4-methylthiophenylazo]benzoic acid with perylene. This shows that 4-[4-methylthiophenylazo]benzoic acid can be used as the quencher when the fluorophore is perylene.

2. Confirmation of quenching-emission with perylene-anthraquinone MB1-C3-PE-Q
The following table shows fluorescent strength at 460 nm from perylene excited at 425 nm before and after addition of MHt to MB1-C3-PE-Q.

**[Table 11]**

| °C | Before MB-t addition | After MB-t addition | Fluorescent strength change before and after MB-t addition: Signal strength ratio |
|---|---|---|---|
| 0 | 2.3 | 90.8 | 36.3 |
| 10 | 2.6 | 84.3 | 32.4 |
| 20 | 2.9 | 77.2 | 26.6 |
| 30 | 3.5 | 69.5 | 20 |
| 40 | 4.4 | 60.5 | 13.8 |
| 50 | 6.7 | 50.5 | 7.5 |
| 60 | 11.7 | 22.3 | 1.9 |

### Fluorescence measurement conditions

| | |
|---|---|
| Fluorescence spectrometer: | JASCO FP-6500 |
| Excitation bandwidth: | 3 mn |
| Fluorescence bandwidth: | 3 nm |
| Response: | 0.1 sec |
| Sensitivity: | Middle |
| Scanning speed: | 200 nm/min |

### MB2-C3-PE-Q

The following table shows the fluorescent strength from at 460 nm from perylene excited at 425 nm before and after addition of MBt to MB2-C3-PE-Q.

**[Table 12]**

| °C | Before MB-t addition | After MB-t addition | Fluorescent strength change before and after MB-t addition: Signal strength ratio |
|---|---|---|---|
| 0 | 4.7 | 90.4 | 19.2 |
| 10 | 4.8 | 86.7 | 18.1 |
| 20 | 5.3 | 81.7 | 15.4 |
| 30 | 6.6 | 75.8 | 11.5 |
| 40 | 8.9 | 69.1 | 7.8 |
| 50 | 14 | 59.8 | 4.3 |
| 60 | 21.8 | 30.8 | 1.4 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

### MB3-C3-PE-Q

The following table shows fluorescent strength at 460 nm from perylene excited at 425 nm before and after addition of MHt to MB3-C3-PE-Q.

**[Table 13]**

| °C | Before MB-t addition | After MB-t addition | Fluorescent strength change before and after MB-t addition: Signal strength ratio |
|---|---|---|---|
| 0 | 0.99 | 74.8 | 75.6 |
| 10 | 1 | 63 | 63 |
| 20 | 0.98 | 56.4 | 57.6 |
| 30 | 1.1 | 47.5 | 43.2 |
| 40 | 1.3 | 36.8 | 28.3 |
| 50 | 1.9 | 21 | 11.1 |
| 60 | 2.8 | 3.5 | 1.3 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

As shown in Tables 11 to 13, quenching-emission behavior attributable to supplying the target nucleic acid was confirmed when anthraquinone was used with perylene. This shows that anthraquinone can be used as the quencher when perylene is the fluorophore. In particular, as shown in Table 13, stable quenching and strong emission were confirmed when two each of perylene and anthraquinone were provided on the paired stem strands of the molecular beacon. It can be seen from these results that quenching when the beacon is shut contributes to an improved signal strength ratio. It is thought that fluorescence quenching is attributable not only to the perylene and anthraquinone making up a pair, but also to movement of electrons via natural base pairs to anthraquinone not forming the pair. That is, the two perylene-anthraquinone pairs introduced into the stem do not function independently, but contribute synergistically to stable quenching.

### 3. Confirmation of quenching-emission with Cy3-MR

### MB1-C3-Cy3-MR

The following table shows fluorescent strength at 563 nm from Cy3 excited at 546 nm before and after addition of MBt to MB1-C3-Cy3-MR.

**[Table 14]**

| °C | Before MB-t addition | After MB-t addition | Fluorescent strength change before and after MB-t addition: Signal strength ratio |
|---|---|---|---|
| 0 | 11 | 227.5 | 20.1 |
| 10 | 8.6 | 178.9 | 20.8 |
| 20 | 7.3 | 135.6 | 18.6 |
| 30 | 6.5 | 100.4 | 15.4 |
| 40 | 6.7 | 71.4 | 10.7 |
| 50 | 8.4 | 47.3 | 5.6 |
| 60 | 14.5 | 20.2 | 1.4 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

### MB2-C3-Cy3-MR

The following table shows fluorescent strength at 563 nm from Cy3 excited at 546 nm before and after addition of MBt to MB2-C3-Cy3-MR.

**[Table 15]**

| °C | Before MB-t addition | After MB-t addition | Fluorescent strength change before and after MB-t addition: Signal strength ratio |
|---|---|---|---|
| 0 | 39.9 | 145.4 | 3.6 |
| 10 | 33 | 114.5 | 3.5 |
| 20 | 26.7 | 90.3 | 3.4 |
| 30 | 22.5 | 71.8 | 3.2 |
| 40 | 21.2 | 57 | 2.7 |
| 50 | 24.2 | 42.7 | 1.8 |
| 60 | 27.8 | 28 | 1 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

As shown in Tables 14 and 15, quenching-emission behavior attributable to supplying the target nucleic acid was confirmed using methyl red with Cy3. This shows that methyl red can be used as a quencher when the fluorophore is Cy3.

### [Example 8]

### Synthesis of amidite monomer containing MRNO2

An amidite monomer containing MRNO2 was synthesized according to the following scheme. 4-amino-3-nitrobenzoic acid was dissolved in a mixed solvent of acetic acid and water, and an acidic hydrochloric acid solution of sodium nitrite was added and agitated at 15 to 20°C for 15 minutes. Ice water was then added to obtain an aqueous solution of diazonium salt. An acidic hydrochloric acid solution of N,N-dimethylaniline was then dripped into this aqueous diazonium salt solution on an ice bath with the temperature maintained at 0 to 5°C, to obtain 4'-dimethylamino-2-nitroazobenzene-4-carboxylic acid (6-1). Next, an amidite monomer (6-2) was obtained according to the methods described in Nature Protocols 2007, Vol. 2, pp. 203-212, using 4'-dimethylamino-2-nitroazobenzene-4-carboxylic acid (6-1) instead of azobenzene.

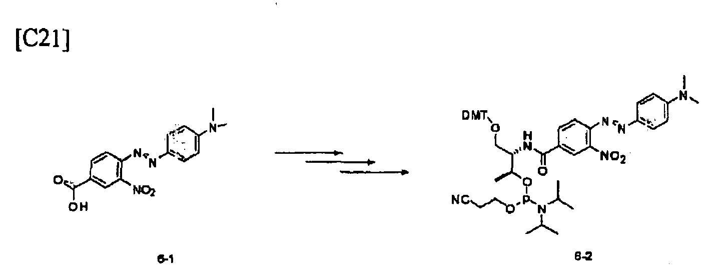

Next, the molecular beacon-type oligonucleotide probes shown in Table 16 below were synthesized. The various molecular beacons were synthesized in accordance with the methods described in Example 4 using the various amidite monomers, fluorophores and quenchers synthesized or provided in Examples 1 to 3. The various sequences are also shown.

**[Table 16]**

| Beacon type | Sequence | X | R | Y | R' |
|---|---|---|---|---|---|
| MBt | MBt | | | | |
| MBt2 | MBt2 | | | | |
| MB1-C3-MRNO2 | MB1 | C3 | Cy3 | C3 | MRNO2 |
| MB2-C3-MRNO2 | MB2 | C3 | Cy3 | C3 | MRNO2 |

The abbreviations in Table 16 are the same as those in drawings below. The abbreviations used in Tables 2 and 8 are also exactly the same as in the drawings below.

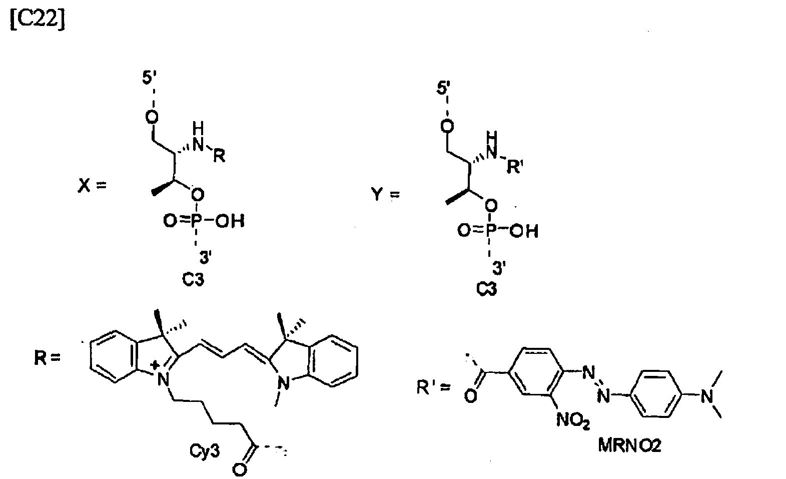

| | |
|---|---|
| MB1: | 5'-TG-X-GTCCTTGAG-AAAGGGCGAC-Y-CA-3' |
| MB2: | 5'-TGG-X-TCCTTGAG-AAAGGGCGA-Y-CCA-3' |
| MBt: | 3'-ACCAGGAACTCTTTCCCG-5' |
| MBt2: | 3'-GAACTCTTTCCCGCTGGT-5' |

Changes in fluorescent strength before and after addition of the target nucleic acid MBt or MBt2 were observed for these beacons. The solution conditions for fluorescent spectrum measurement were as follows. The results are shown in Tables 17 to 20.

| | |
|---|---|
| Beacon concentration: | 0.2 µmol/l |
| Target nucleic acid Nb concentration: | 0.4 µmol/l |
| Sodium chloride: | 0.1 µmol/l |
| Phosphate buffer: | 10 mmol/l (pH 7.0) |

### MB1-C3-Cy3-MRNO2

The following table shows fluorescent strength at 566 nm from Cy3 excited at 546 nm before and after addition of MB-t to MB1-C3-Cy3-MRNO2.

**[Table 17]**

| °C | Before MB-t addition | After MB-t addition | Fluorescent strength change before and after MB-t addition: Signal strength ratio |
|---|---|---|---|
| 0 | 7 | 391 | 56 |
| 10 | 6 | 311 | 52 |
| 20 | 4.8 | 241 | 50 |
| 30 | 4.5 | 178 | 40 |
| 40 | 4.7 | 127 | 27 |
| 50 | 7.4 | 82.7 | 11 |
| 60 | 13.5 | 29.2 | 2.2 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

The following table shows fluorescent strength at 566 nm from Cy3 excited at 546 nm before and after addition of MB-t2 to MB1-C3-Cy3-MRNO2.

**[Table 18]**

| °C | Before MB-t2 addition | After MB-t2 addition | Fluorescent strength change before and after MB-t2 addition: Signal strength ratio |
|---|---|---|---|
| 0 | 7 | 483 | 69 |
| 10 | 6 | 417 | 70 |
| 20 | 4.8 | 348 | 73 |
| 30 | 4.5 | 279 | 62 |
| 40 | 4.7 | 211 | 45 |
| 50 | 7.4 | 146 | 20 |
| 60 | 13.5 | 48 | 3.6 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

### MB2-C3-Cy3-MRNO2

The following table shows fluorescent strength at 566 nm from Cy3 excited at 546 nm before and after addition of MB-t to MB2-C3-Cy3-MRNO2.

**[Table 19]**

| °C | Before MB-t addition | After NiB-t addition | Fluorescent strength change before and after MB-t addition: Signal strength ratio |
|---|---|---|---|
| 0 | 18.2 | 261 | 14.3 |
| 10 | 15.6 | 206 | 13.2 |
| 20 | 13.4 | 164 | 12.2 |
| 30 | 11.4 | 132 | 11.6 |
| 40 | 11.7 | 105 | 8.97 |
| 50 | 14.3 | 73.6 | 5.15 |
| 60 | 16.2 | 38.2 | 2.36 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

The following table shows fluorescent strength at 566 nm from Cy3 excited at 546 nm before and after addition of MB-t2 to MB2-C3-Cy3-MRNO2.

**[Table 20]**

| °C | Before MB-t2 addition | After MB-t2 addition | Fluorescent strength change before and after MB-t2 addition: Signal strength ratio |
|---|---|---|---|
| 0 | 18.2 | 459 | 25.2 |
| 10 | 15.6 | 392 | 25.1 |
| 20 | 13.4 | 329 | 24.6 |
| 30 | 11.4 | 263 | 23.1 |
| 40 | 11.7 | 204 | 17.4 |
| 50 | 14.3 | 145 | 10.1 |
| 60 | 16.2 | 62 | 3.83 |

### Fluorescence measurement conditions

| | |
|---|---|
| Fluorescence spectrometer: | JASCO FP-6500 |
| Excitation bandwidth: | 3 nm |
| Fluorescence bandwidth: | 3 nm |
| Response: | 0.1 sec |
| Sensitivity: | Middle |
| Scanning speed: | 200 nm/min |

As shown in Tables 17 to 20, quenching-emission behavior attributable to supplying the target nucleic acid was confirmed using MRNO2 with Cy3. This shows that MRNO2 can be used as the quencher when the fluorophore is Cy3. The maximum absorption wavelength of Cy3 is 546 nm while that of MRNO2 is 513 nm, and the difference is 33 nm.
Quenching-emission behavior was confirmed whether the target nucleic acid formed a double strand with the fluorescent dye-side strand of the beacon as in Tables 17 and 19, or with the quencher side of the beacon as in Tables 18 and 20.

### Sequence Table Free Text

Sequence Nos. 1 to 8: Synthetic oligonucleotides

## Claims

1. An oligonucleotide probe capable of forming a stem and loop, comprising:
at least one fluorophore that is located between adjacent nucleotides in the stem and is linked to a unit represented by the following Formula (1): 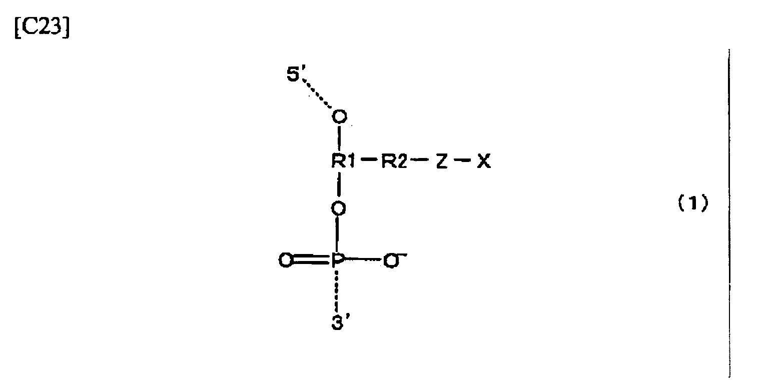 (in the formula, X represents the fluorophore, R1 represents an optionally substituted C₂ or C₃ alkylene chain, R2 represents an optionally substituted C₀₋₂ alkylene chain, and Z represents a direct bond or linker); and
at least one quencher that is located at a site capable of pairing up with the at least one fluorophore located between the adjacent nucleotides in the stem and is linked to a unit represented by the following Formula (2): 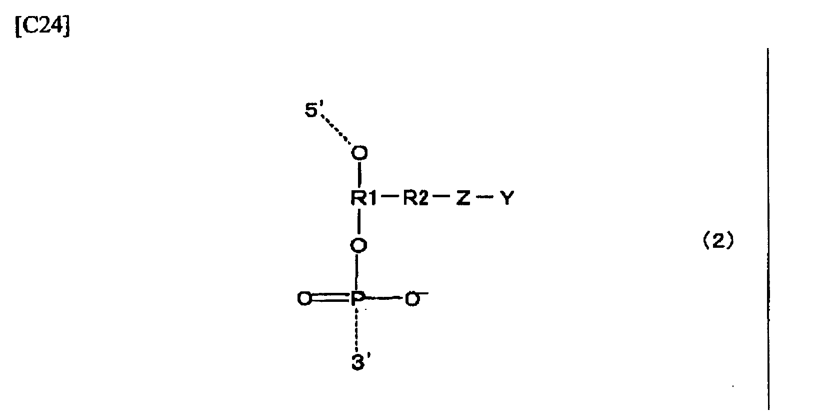 (in the formula, Y represents the quencher, R1 represents an optionally substituted C₂ or C₃ alkylene chain, R2 represents an optionally substituted C₀₋₂ alkylene chain, and Z represents a direct bond or linker).

2. The probe according to Claim 1, wherein the fluorophore is located within a nucleotide strand constituting a base sequence that hybridizes specifically with a target nucleic acid.

3. The probe according to Claim 1 or 2, wherein the fluorophore is any one selected from the group consisting of cyanine dyes, merocyanine dyes, condensed aromatic ring dyes, xanthene dyes, coumarin dyes and acridine dyes, and the quencher is any one selected from the group consisting of azo dyes.

4. The probe according to Claim 3, wherein the fluorophore is any one selected from Cy3, Cy5, thiazole orange, oxazole yellow, perylene, fluorescein, rhodamine, tetramethyl rhodamine, Texas red, coumarin and acridine, and the quencher is any one selected from methyl red, azobenzene and methylthioazobenzene.

5. The probe according to Claim 3, wherein the fluorophore is a condensed aromatic ring dye, and the quencher is anthraquinone or a derivative thereof.

6. The probe according to Claim 5, wherein the fluorophore is perylene, and the quencher is anthraquinone.

7. The probe according to any one of Claims 1 to 6, capable of forming one stem and one loop.

8. An immobilized probe body for detecting a target nucleic acid, comprising:
a solid-phase carrier; and
the oligonucleotide probe according to any one of Claims 1 to 7 supported on the solid-phase carrier and having a base sequence capable of hybridizing specifically with at least part of the target nucleic acid.

9. The immobilized body according to Claim 8, wherein the solid-phase carrier is a plate.

10. The immobilized body according to Claim 8 or 9, comprising a plurality of oligonucleotide probes capable of detecting a plurality of the target nucleic acids, respectively.

11. The immobilized body according to any one of Claims 8 to 10, which is used for detecting SNPs or gene variants.

12. A method for detecting a target nucleic acid, comprising:
using the oligonucleotide probe according to any one of Claim 1 to 7 having a base sequence capable of hybridizing specifically with at least part of the target nucleic acid, to detect a signal based on the fluorophore of the oligonucleotide probe.

13. The method according to Claim 12, using an immobilized body having a solid-phase carrier supporting a plurality of the oligonucleotide probes capable of pairing and hybridizing with a plurality of target nucleic acids.
